# EUROPÄISCHE PATENTSCHRIFT

(11) **EP 0 944 633 B1**
(45) Veröffentlichungstag und Bekanntmachung des Hinweises auf die Patenterteilung: **10.10.2007**
(21) Anmeldenummer: 97952026.9
(22) Anmeldetag: 01.12.1997
(51) Int. Cl.: C07D 491/10, C07D 493/10, C07D 495/10, A01N 43/90, C07F 9/6561, C07D 309/10, C07D 309/14, C07D 309/08

(54) **SUBSTITUIERTE PHENYLKETOENOLE UND IHRE VERWENDUNG ALS SCHÄDLINGSBEKÄMPFUNGSMITTEL**
SUBSTITUTED PHENYLKETOENOLS AND THEIR USE AS PESTICIDES AND HERBICIDES
PHENYLCETOENOLS SUBSTITUES ET LEUR UTILISATION COMME PESTICIDES ET HERBICIDES

(30) Priorität: 12.12.1996 DE 19651686
(43) Veröffentlichungstag der Anmeldung: 29.09.1999
(73) Patentinhaber: Bayer CropScience AG, 40789 Monheim (DE)
(72) Erfinder: HAGEMANN, Hermann, D-51375 Leverkusen (DE); FISCHER, Reiner, D-40789 Monheim (DE); BRETSCHNEIDER, Thomas, D-53797 Lohmar (DE); ERDELEN, Christoph, D-42799 Leichlingen (DE); WACHENDORFF-NEUMANN, Ulrike, D-56566 Neuwied (DE); DAHMEN, Peter, D-41470 Neuss (DE); DOLLINGER, Markus, D-51381 Leverkusen (DE); GRAFF, Alan, D-51061 Köln (DE); ANDERSCH, Wolfram, D-51469 Bergisch Gladbach (DE)
(86) Internationale Anmeldenummer: PCT/EP1997/006708
(87) Internationale Veröffentlichungsnummer: WO 1998/025928

(56) Entgegenhaltungen:
- EP-A- 0 521 334
- EP-A- 0 647 637
- WO-A-95/26954
- DE-A- 4 216 814
- DE-A- 4 410 420
- DE-A- 4 415 334
- DE-A- 4 425 617
- DE-A- 4 431 730
- DE-A- 19 540 736
- DE-A- 19 543 864
- DE-A- 19 545 467

## Beschreibung

Die Erfindung betrifft neue phenylsubstituierte cyclische Ketoenole, mehrere Verfahren und Zwischenprodukte zu ihrer Herstellung und ihre Verwendung als Schädlingsbekämpfungsmittel und Herbizide.

Es ist bereits bekannt geworden, daß bestimmte phenylsubstituierte cyclische Ketoenole als Insektizide, Akarizide und/oder Herbizide wirksam sind.

Bekannt sind 1H-Arylpyrrolidin-dion-Derivate (EP-A-456 063, EP-A-521 334, EP-A-596 298, EP-A-613 884, EP-A-613 885, DE 44 40 594, WO 94/01 997, WO 95/01 358, WO 95/20 572, EP-A-668 267, WO 95/26 954, WO 96/25395 und WO 96/35 664) mit insektiziden, akariziden und gegebenenfalls herbiziden Wirkungen.

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 96/25395, WO 96/20196 und der oben genannte noch nicht offengelegten Patenanmeldung bekannt. Auch 3-Aryl-Δ³-di-hydrothiophen-on-Derivate sind bekannt (WO 95/26 345, WO 96/25395 und WO 96/35664).

Die akarizide und insektizide Wirksamkeit und/oder Wirkungsbreite und/oder die Pflanzenverträglichkeit dieser Verbindungen, insbesondere gegenüber Kulturpflanzen, ist jedoch nicht immer ausreichend.

Es wurden nun neue Verbindungen der Formel (I) 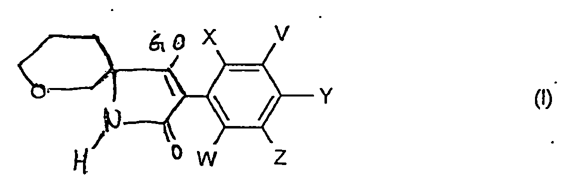 gefünden,
in welcher
- V: für Wasserstoff: Halogen, C₁-C₆-Alkyl oder C₁-C₆-Alkoxy steht,
- W: für Wasserstoff, Nitro, Cyano, Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht,
- X: für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy, Cyano, Nitro oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht,
- Y: für Wasserstoff, Halogen, C₁-C₆-Alkyl, C₁-C₆-Alkoxy, C₁-C₄-Halogenalkyl, C₁-C₄-Halogenalkoxy, Cyano oder Nitro steht,
- Z: für Wasserstoff, Halogen, C₁-C₆-Alkyl, C₁-C₆-Alkoxy, C₁-C₄-Halogenalkyl, C₁-C₄-Halogenalkoxy, Hydroxy, Cyano, Nitro oder für jeweils gegebenenfalls durch Halogen, C₁-C₄-Alkyl C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht oder steht,
- G: für Wasserstoff (a) oder für eine der Gruppen 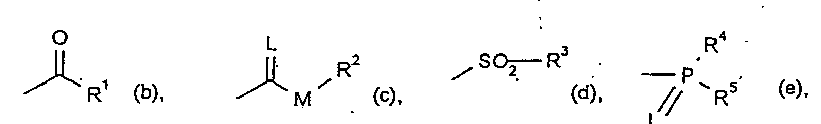 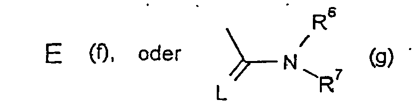 steht,
in welchen
E für ein Metallionäquivalent oder ein Ammoniumion steht,
L für Sauerstoff oder Schwefel steht und
M für Sauerstoff oder Schwefel steht.

- R¹: für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl steht, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl-oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
- R²: für jeweils gegebenenfalls durch Halogen oder Cyano substituiertes C₁-C₂₀-Alkyl, C₂-C₂₀-Alkenyl, C₁- C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
- R³: für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
- R⁴ und R⁵: unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
- R⁶ und R⁷: unabhängig voneinander für Wasserstoff für jeweils gegebenenfalls durch Halogen oder Cyano substituiertes C₁-C₈-Alkyl C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), 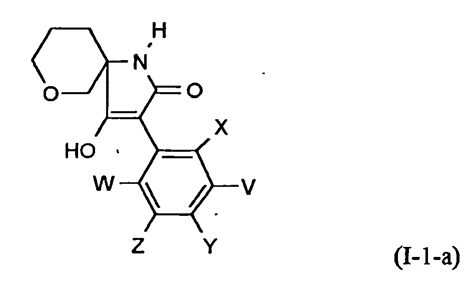 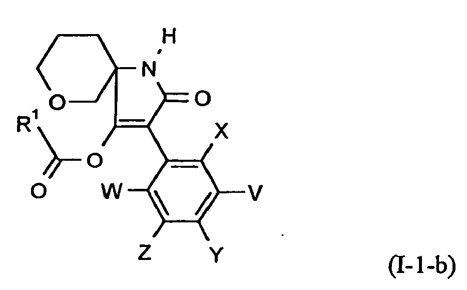 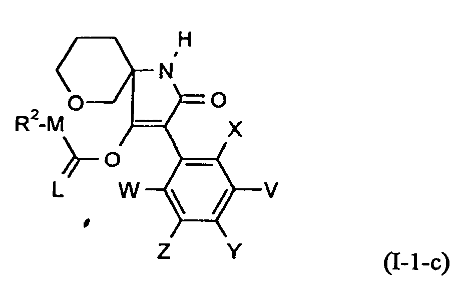 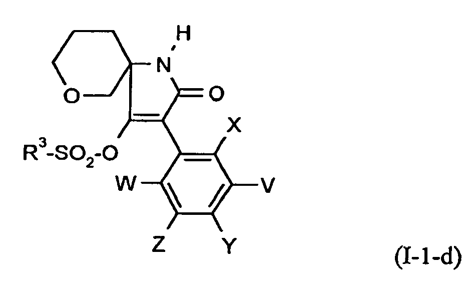 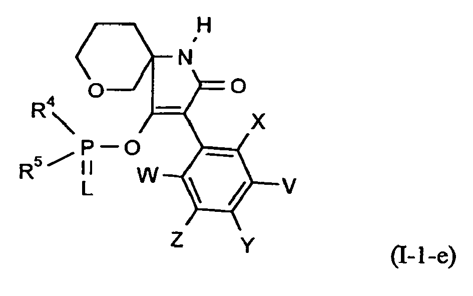 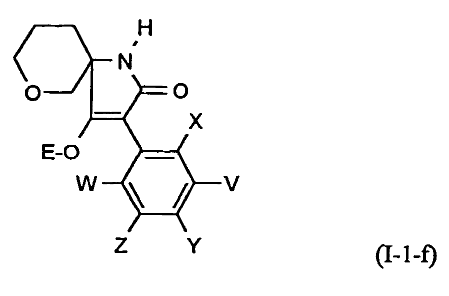 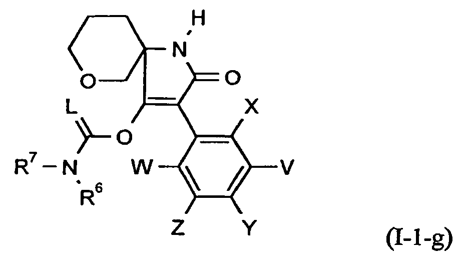 worin
E, L, M, V, W, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach den im folgenden beschriebenen Verfahren erhält:
(A) Man erhält Verbindungen der Formel (I-1-a) 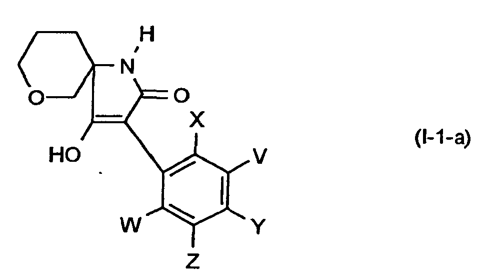 in welcher
   V, W, X, Y und Z die oben angegebenen Bedeutungen haben,
   wenn man
   Verbindungen der Formel (II) 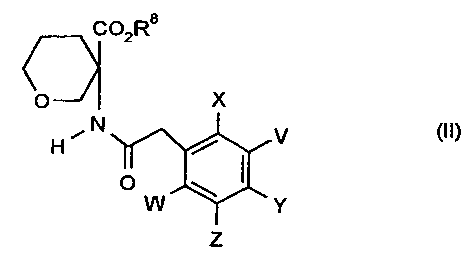 in welcher
   V, W, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert. Außerdem wurde gefunden,
(D) daß man die Verbindungen der oben gezeigten Formel (I-1-b), in welchen R¹, V, W, X, Y und Z die oben angebenen Bedeutungen haben, erhält, wenn man Verbindungen der oben gezeigten Formel (I-1-a), in welchen V, W, X, Y und Z die oben angegebenen Bedeutungen haben, jeweils
   α) mit Säurehalogeniden der Formel (V) 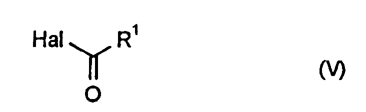 in welcher
      - R¹: die oben angegebene Bedeutung hat und
      - Hal: für Halogen (insbesondere Chlor oder Brom) steht
      oder
   β) mit Carbonsäureanhydriden der Formel (VI)

      R¹-CO-O-CO-R¹ (VI)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(E) daß man die Verbindungen der oben gezeigten Formel (I-1-c), in welchen R², V, W, M, X, Y und Z die oben angegebenen Bedeutungen haben und L für Sauerstoff steht, erhält, wenn man Verbindungen der oben gezeigten Formel (I-1-a), in welchen V, W, X, Y und Z die oben angegebenen Bedeutungen haben, jeweils
   mit Chlorameisensäureestern oder Chlorameisensäurethioestern der Formel
   (VII)

   R²-M-CO-Cl (VII)

   in welcher
   R² und M die oben angegebenen Bedeutungen haben,
   gegebenenfalls in Gegenwart eines Verdünnungsmittels und gegebenenfalls in
   Gegenwart eines Säurebindemittels umsetzt;
(F) daß man Verbindungen der oben gezeigten Formel (I-1-c), in welchen R², V, W, M, X, Y und Z die oben angegebenen Bedeutungen haben und L für Schwefel steht, erhält, wenn man Verbindungen der oben gezeigten Formel (I-1-a), in welchen V, W, X, Y und Z die oben angegebenen Bedeutungen haben, jeweils
   mit Chlormonothioameisensäureestern oder Chlordithioameisensäureestern der Formel (VIII) 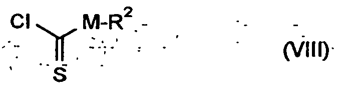 in welcher
   M und R² die oben angegebenen Bedeutungen haben,
   gegebenenfalls in Gegenwart eines Verdünnungsmittels und gegebenenfalls in Gegenwart eines Säurebindemittels umsetzt,
(G) daß man Verbindungen der oben gezeigten Formel (I-1-d), in welchen R³, V, W, X, Y und Z die oben angegebenen Bedeutungen haben, erhält, wenn man Verbindungen der oben gezeigten Formel (I-1-a), in welchen V, W, X, Y und Z die oben angegebenen Bedeutungen haben, jeweils
   mit Sulfonsäurechloriden der Formel (IX)

   R³-SO₂-Cl (IX)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(H) daß man Verbindungen der oben gezeigten Formel (I-1-e), in welchen L, R⁴, R⁵, V, W, X, Y und Z die oben angegebenen Bedeutungen haben, erhält, wenn man Verbindungen der oben gezeigten Formel (I-1-a), in welchen V, W, X, Y und Z die oben angegebenen Bedeutungen haben, jeweils
   mit Phosphorverbindungen, der Formel (X) 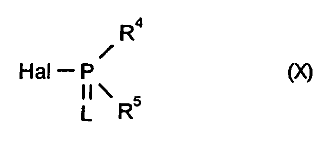 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(I) daß man Verbindungen der oben gezeigten Formel (I-1-f), in welchen E, V, W, X, Y und Z die oben angegebenen Bedeutungen haben, erhält, wenn man Verbindungen der Formel (I-1-a), in welchen V, W, X, Y und Z die oben angegebenen Bedeutungen haben, jeweils
   mit Metallverbindungen oder Aminen der Formeln (XI) oder (XII)

   Me(OR¹⁰)ₜ (XI)

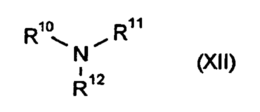

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoffoder Alkyl (bevorzugt C₁-C₈-Alkyl) stehen,
   gegebenenfalls in Gegenwart eines Verdünnungsmittels umsetzt,
(J) daß man Verbindungen der oben gezeigten Formel (I-1-g), in welchen L, R⁶, R⁷, V, W, X, Y und Z die oben angegebenen Bedeutungen haben, erhält, wenn man Verbindungen der oben gezeigten Formel (I-1-a),
   in welchen V, W, X, Y und Z die oben angegebenen Bedeutungen haben, jeweils
   α) mit Isocyanaten oder Isothiocyanaten der Formel (XIII)

      R⁶-N=C=L (XIII)

      in welcher
      R⁶ und L die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XIV) 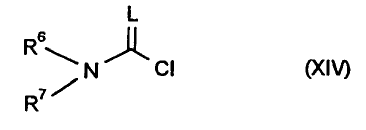 in welcher
      L, R⁶ und R⁷ die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen und darüber hinaus häufig sehr gut pflanzenverträglich, insbesondere gegenüber Kulturpflanzen, sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- V: steht besonders bevorzugt für Wasserstoff, Fluor, Chlor, Brom, C₁-C₄-Alkyl oder C₁-C₄-Alkoxy.
- W: steht besonders bevorzugt für Wasserstoff, Nitro, Cyano, Fluor, Chlor, Brom, C₁-C₄-Alkyl, C₁-C₄-Alkoxy, C₁-C₂-Halogenalkyl, C₁-C₂-Halogenalkoxy oder für jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy oder Benzyloxy.
- X: steht besonders bevorzugt für Fluor, Chlor, Brom, C₁-C₄-Alkyl C₁-C₄-Alkoxy, C₁-C₂-Halogenalkyl, C₁-C₂-Halogenalkoxy, Cyano, Nitro oder jeweils gegebenenfalls durch Fluor, Chlor, Brom, C₁-C₄-Alkyl, C₁-C₄-Alkoxy, C₁-C₂-Halogenakyl C₁-C₂-Halogenalkoxy, Nitro oder Cyano substituiertes Phenyl, Phenoxy oder Benzyloxy.
- Y: steht besonders bevorzugt für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano oder Nitro.
- Z: steht besonders bevorzugt für Wasserstoff Fluor, Chlor, Brom, C₁-C₄-Alkyl, C₁-C₄-Alkoxy, C₁-C₂-Halogenalkyl, C₁-C₂-Halogenalkoxy, Hydroxy, Cyano, Nitro oder für jeweils gegebenefalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy oder Benzyloxy, oder .
- G: steht besonders bevorzugt für Wasserstoff (a) oder für eine der Gruppen 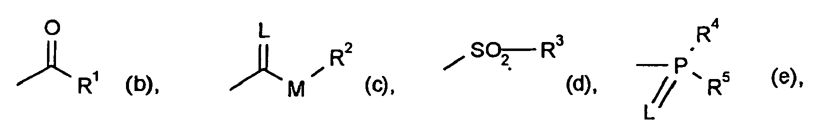 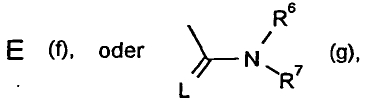 (insbesondere für (a), (b) oder (c))
in welchen
E für ein Metallionäquivalent oder ein Ammoniumion steht,
L für Sauerstoff oder Schwefel steht und
M für Sauerstoff oder Schwefel steht.

- R¹: steht besonders bevorzugt für jeweils gegebenenfalls durch Fluor oder Chlor substituiertes C₁-C₁₆-Alkyl C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl, für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls durch Fluor oder Chlor substituiertes C₁-C₆-Alkyl oder für jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder BenzyL
- R⁴ und R⁵: stehen unabhängig voneinander besonders bevorzugt für jeweils gegebenenfalls durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.

- R⁶ und R⁷: stehen unabhängig voneinander besonders bevorzugt für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.
- V: steht ganz besonders bevorzugt für Wasserstoff Fluor, Chlor, Brom, Methyl, Ethyl, Propyl, iso-Propyl, tert.-Butyl, Methoxy, Ethoxy, Propoxy oder iso-Propoxy.
- W: steht ganz besonders bevorzugt für Wasserstoff, Nitro, Cyano, Fluor, Chlor, Brom, Methyl, Ethyl, Propyl, n-Butyl, iso-Propyl, iso-Butyl, Methoxy, Ethoxy, Propoxy, iso-Propoxy, Trifluormethyl, Difluormethoxy, Trifluormethoxy, Phenyl oder Benzyloxy.
- X: steht ganz besonders bevorzugt für Fluor, Chlor, Brom, Methyl, Ethyl, Propyl, Butyl, iso-Butyl, iso-Propyl, Methoxy, Ethoxy, Propoxy, iso-Propoxy, Trifluormethyl, Trifluormethoxy, Difluormethoxy, Cyano, Nitro, Phenyl oder Benzyloxy.
- Y: steht ganz besonders bevorzugt für Wasserstoff, Fluor, Chlor, Brom, Methyl, Ethyl, Propyl, iso-Propyl, n-Butyl, iso-Butyl, tert.-Butyl, Methoxy, Ethoxy, Propoxy, iso-Propoxy, Trifluormethyl, Trifluormethoxy, Difluormethoxy, Cyano oder Nitro.
- Z: steht ganz besonders bevorzugt für Wasserstoff, Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano oder Nitro, oder
- G: steht ganz besonders bevorzugt für Wasserstoff (a) oder für eine der Gruppen 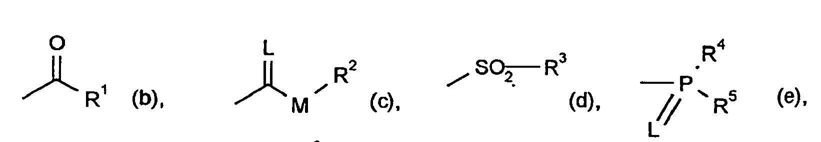 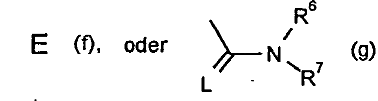 (insbesondere für (a), (b) oder (c))
in welchen
E für ein Metallionäquivalent oder ein Ammoniumion steht,
L für Sauerstoff oder Schwefel steht und
M für Sauerstoff oder Schwefel steht.

- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-akyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl.
- R³: steht ganz besonders bevorzugt für jeweils gegebenenfalls durch Fluor oder Chlor substituiertes Methyl, Ethyl, Propyl, iso-Propyl, n-Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl.
- R⁴ und R⁵: stehen unabhängig voneinander ganz besonders bevorzugt für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, in welchem gegebenenfalls eine Methylengruppe durch Sauerstoff oder Schwefel ersetzt ist.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

**Tabelle 1**

| | | | | |
|---|---|---|---|---|
| 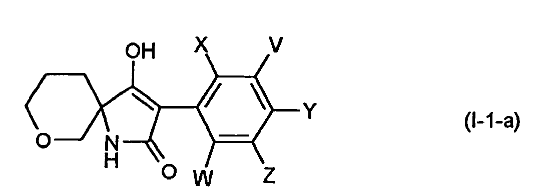 | | | | |

| V | X | W | Y | Z |
|---|---|---|---|---|
| H | Br | H | Cl | H |
| H | Cl | H | Br | H |
| H | Cl | H | Cl | H |
| H | Cl | H | F | H |
| H | F | H | Cl | H |
| H | Cl | H | OCH₃ | H |
| H | Cl | H | CH₃ | H |
| H | OCH₃ | H | Cl | H |
| H | OCH₃ | H | OCH₃ | H |
| H | CH₃ | H | Cl | H |
| H | CH₃ | H | F | H |
| H | CH₃ | H | OCH₃ | H |
| H | CH₃ | H | t-C₄H₉ | H |
| H | CH₃ | H | CH₃ | H |
| H | Cl | Cl | H | H |
| H | Cl | F | H | H |
| H | Cl | OCH₃ | H | H |
| H | Cl | CH₃ | H | H |
| H | Cl | OC₂H₅ | H | H |
| H | OCH₃ | OCH₃ | H | H |
| H | CH₃ | CH₃ | H | H |
| H | Br | CH₃ | Br | H |
| H | Cl | Cl | CH₃ | H |
| H | CH₃ | Br | CH₃ | H |
| H | CH₃ | Cl | CH₃ | H |
| H | CH₃ | OCHF₂ | CH₃ | H |
| H | CH₃ | OCH₂CF₃ | CH₃ | H |
| H | CH₃ | OC₂H₅ | CH₃ | H |
| H | CH₃ | OCH₃ | CH₃ | H |
| H | CH₃ | CHCH | ₃ | H |
| H | Br | Br | CH₃ | H |
| H | Cl | Cl | CH₃ | H |
| H | C₂H₅ | C₂H₅ | Br | H |
| H | CH₃ | CH₃ | Br | H |
| H | CH₃ | CH₃ | OCH₃ | H |
| H | Br | Cl | CH₃ | H |
| H | Br | CH₃ | Cl | H |
| H | Cl | CH₃ | Br | H |
| H | C₂H₅ | Br | CH₃ | H |
| H | CH₃ | O-C₃H₇ | CH₃ | H |
| H | CH₃ | O-Bz* | CH₃ | H |
| H | CH₃ | CH₃ | Cl | H |
| H | CH₃ | Ph* | CH₃ | H |
| H | Cl | H | Cl | Cl |
| H | CH₃ | H | CH₃ | CH₃ |
| H | CH₃ | H | Cl | CH₃ |
| H | Br | H | Cl | CH₃ |
| H | Br | H | CH₃ | CH₃ |
| H | Cl | H | Br | CH₃ |
| H | Cl | H | Cl | CH₃ |
| H | CH₃ | H | Br | CH₃ |
| H | Cl | H | Cl | F |
| H | Cl | H | CH₃ | Cl |
| H | CH₃ | H | H | H |
| H | Cl | H | H | H |
| H | Br | H | H | H |
| H | O-Bz | H | H | H |
| H | CF₃ | H | H | H |
| H | OCH₃ | H | H | H |
| H | CH₃ | CH₃ | CH₃ | CH₃ |
| CH₃ | CH₃ | H | CH₃ | CH₃ |
| CH₃ | CH₃ | CH₃ | H | CH₃ |
| H | CH₃ | CH₃ | CH₃ | F |
| H | CH₃ | CH₃ | CH₃ | Cl |
| H | CH₃ | CH₃ | CH₃ | Br |
| H | CH₃ | CH₃ | H | Cl |
| H | CH₃ | CH₃ | H | Br |
| H | Cl | Cl | H | Br |
| CH₃ | CH₃ | CH₃ | CH₃ | CH₃ |

| | | | | |
|---|---|---|---|---|
| *** Bz = Benzyl; Ph = Phenyl** | | | | |

Verwendet man gemäß Verfahren (A) N-[(4-Chlor-2,6-dimethyl)-phenylacetyl]-3-amino-3-carboxyethyl-tetrahydropyran als Ausgangsstoff, so kann der Verlauf des erfindungsgemäßen Verfahrens durch folgendes Reaktionsschema wiedergegeben werden:

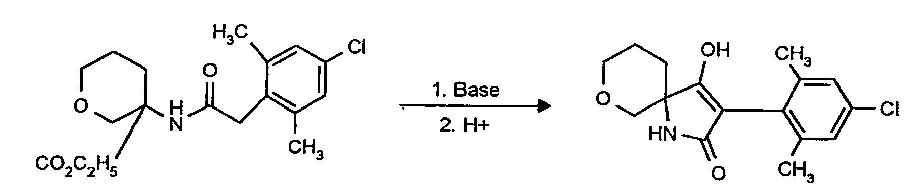

Verwendet man gemäß Verfahren (B) beispielsweise O-[(2-Chlor-6-methyl)-phenylacetyl]-3-hydroxy-3-carboxyethyl-tetrahydropyran, so kann der Verlauf des erfindungsgemäßen Verfahrens durch folgendes Reaktionsschema wiedergegeben werden:

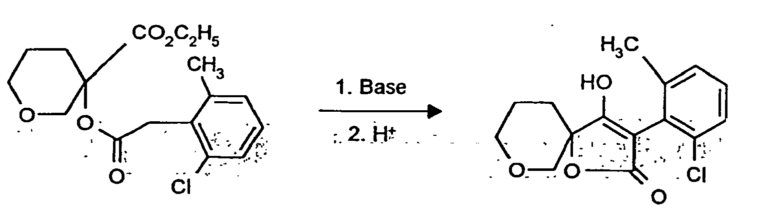

Verwendet man gemäß Verfahren (c) beispielsweise 2-[(2-Chlor-4,6-dimethyl)-phenyl]-4-(4-methoxy)-benzylmercapto-4,4-methylenoxypropyl-3-oxo-valeriansäure-ethylester, so kann der Verlauf des erfindungsgemäßen Verfahrens durch folgendes Reaktionsschema wiedergegeben werden:

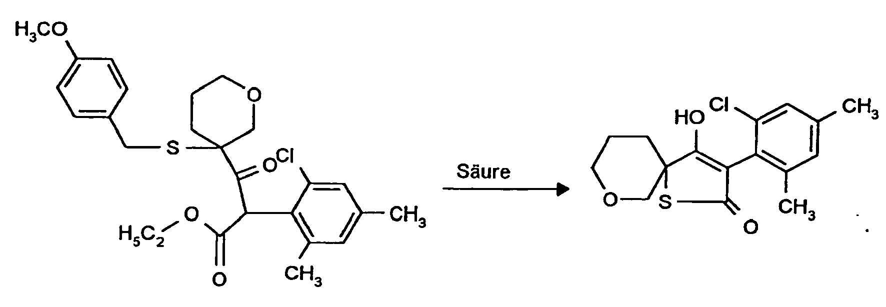

Verwendet man gemäß Verfahren (Dα) 3-[(2-Chlor-4-methyl)-phenyl]-5,5-methylen-oxypropyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

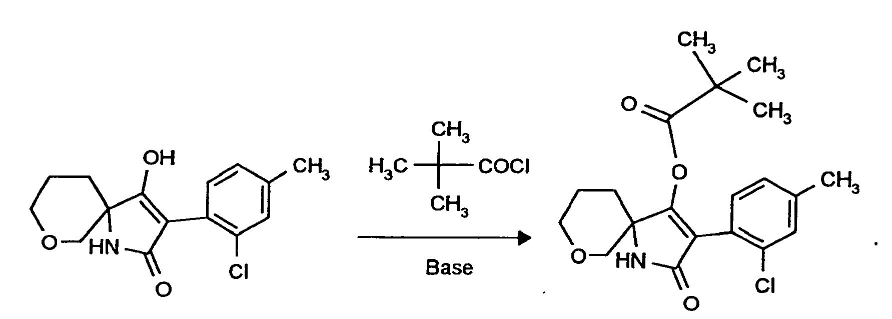

Verwendet man gemäß Verfahren (D) (Variante β) beispielsweise 3-[(2,4-Dichlor)-phenyl]-4-hydroxy-5,5-methylenoxypropyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

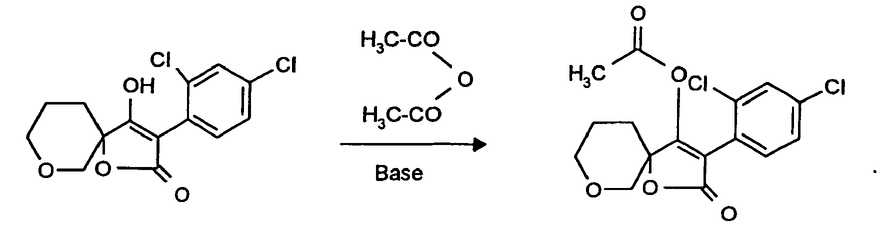

Verwendet man gemäß Verfahren (E) 8-[(2,4-Dichlor)-phenyl]-5,5-methylenoxypropyl-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

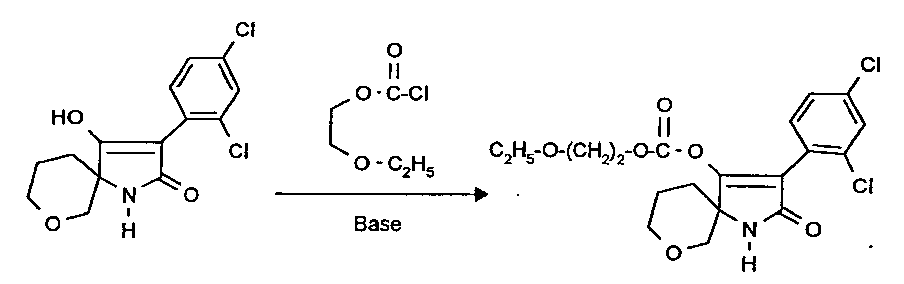

Verwendet man gemäß Verfahren (F) beispielsweise 3-[(2,6-Dibrom-4-methyl)-phenyl]-4-hydroxy-5,5-methylenoxypropyl-Δ³-dihydrofuran-2-on und Chlormonothioameisensäuremethylester als Ausgangsprodukte, so kann der Reaktionsverlauf folgendermaßen wiedergegeben werden:

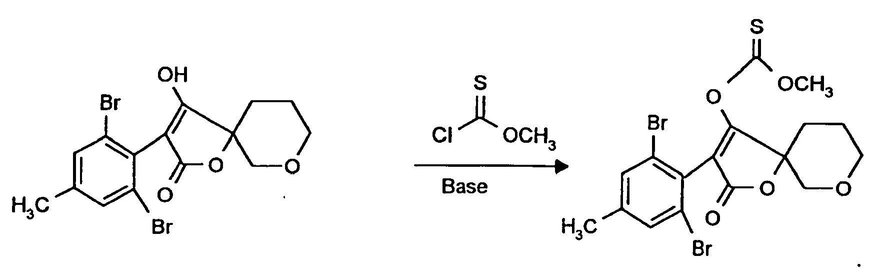

Verwendet man gemäß Verfahren (G) 2-[(2,4,6-Trimethyl)-phenyl]-5,5-methylenoxypropyl-pyrrolidin-2,4-dion und Methansulfonsäurechlorid als Ausgangsprodukt, so kann der Reaktionsverlauf durch folgendes Reaktionsschema wiedergegeben werden:

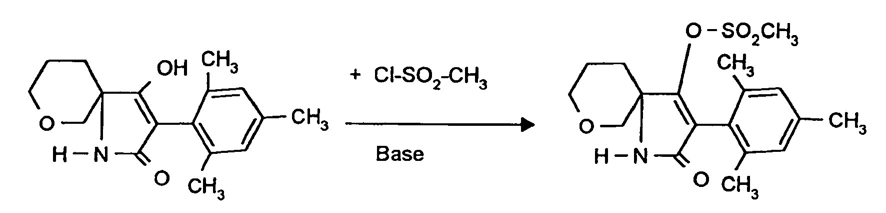

Verwendet man gemäß Verfahren (H) beispielsweise 2-[(4-Brom-2-chlor-6-methyl)-phenyl]-4-hydroxy-5,5-methylenoxypropyl-Δ³-dihydrofuran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

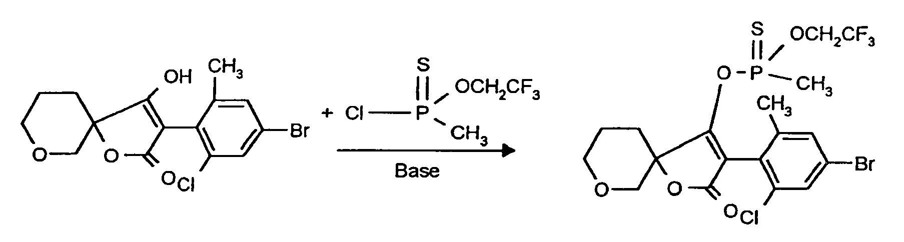

Verwendet man gemäß Verfahren (I) 3-[(2,4-Dichlor)-6-methylphenyl]-5,5-methylenoxypropyl-pyrrolidin-2,4-dion und NaOH als Komponenten, so kann der Verlauf des erfindungsgemäßen Verfahrens durch folgendes Reaktionsschema wiedergegeben werden:

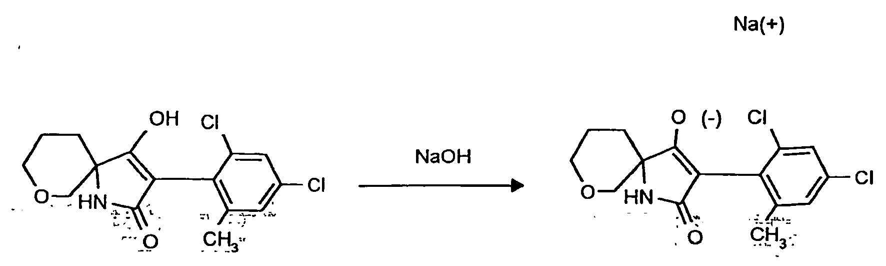

Verwendet man gemäß Verfahren (J) (Variante α) beispielsweise 3-[(2-Chlor-4-brom-5-methyl)-phenyl]-4-hydroxy-5,5-methylenoxypropyl-Δ³-dihydro-furan-2-on und Ethylisocyanat als Ausgangsprodukte, so kann der Reaktionsverlauf durch folgendes Reaktionsschema wiedergegeben werden:

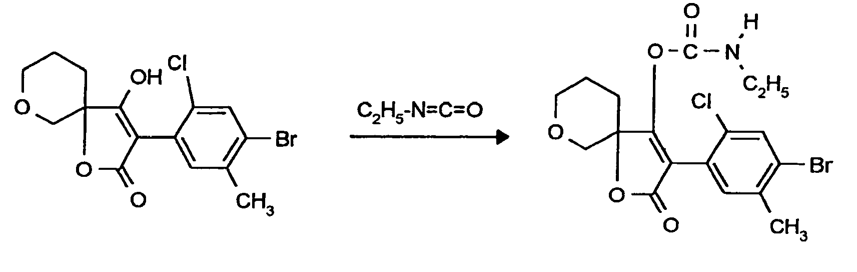

Verwendet man gemäß Verfahren (J) (Variante β) 3-[(2-Chlor-4,6-dimethyl)-phenyl]-5,5-methylenoxypropyl-pyrrolidin-2,4-dion und Dimethylcarbamidsäurechlorid als Ausgangsprodukte, so kann der Reaktionsverlauf durch folgendes Schema wiedergegeben werden:

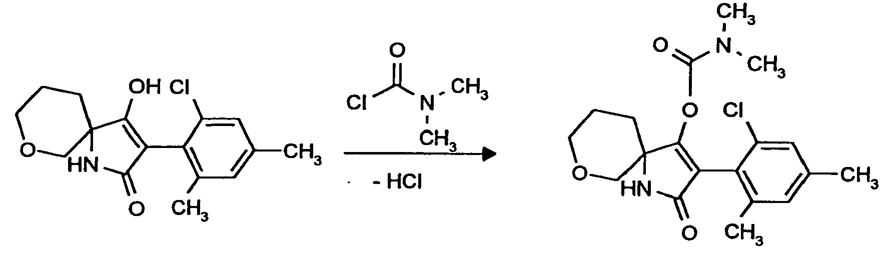

Die beim erfindungsgemäßen Verfahren (A) als Ausgangsstoffe benötigten Verbindungen der Formel (II) 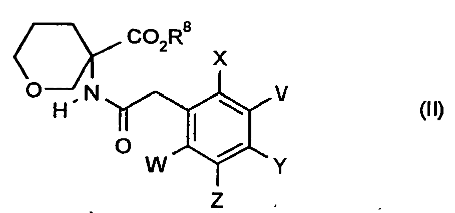 in welcher
- V, W, X, Y, Z und R⁸: die oben angegebenen Bedeutungen haben,
sind neu.

Man erhält die Acylaminosäureester der Formel (II) beispielsweise, wenn man Aminosäurederivate der Formel (XV) 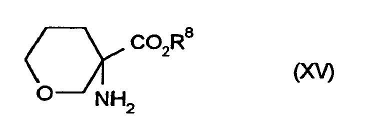 in welcher
- R⁸: die oben angegebene Bedeutung hat,
mit substituierten Phenylessigsäurehalogeniden der Formel (XVI) 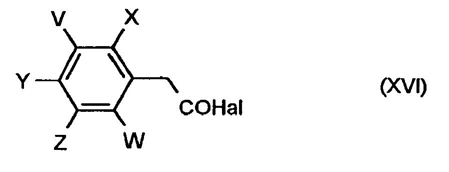 in welcher
- V, W, X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XVII) 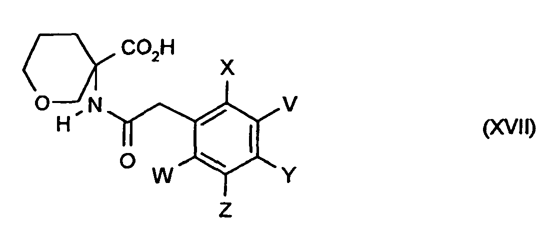 in welcher
- V, W, X, Y und Z: die oben angegebenen Bedeutungen haben,
verestert (Chem. Ind. (London) 1568 (1968)).

Die Verbindungen der Formel (XVII) 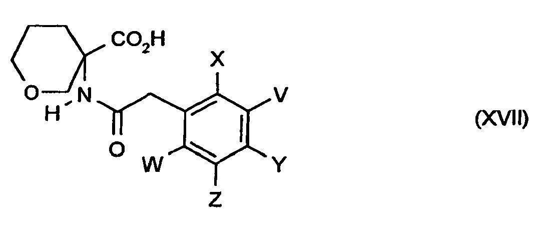 in welcher
- V, W, X, Y und Z: die oben angegebenen Bedeutungen haben,
sind neu.

Man erhält die Verbindungen der Formel (XVII) beispielsweise, wenn man 3-Aminotetrahydropyran-3-carbonsäure der Formel (XVIII) 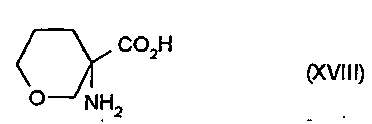 mit substituierten Phenylessigsäurehalogeniden der Formel (XVI) 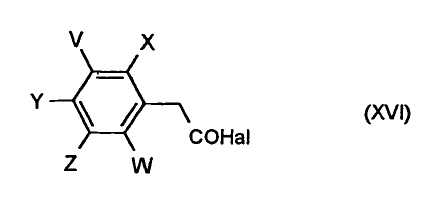 in welcher
- V, W, X, Y und Z: die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XVI) sind teilweise neu und lassen sich nach bekannten Verfahren herstellen (vgl. z.B. WO 97/02 243, WO 97/01 535 und DE-196 13 171).

Man erhält die Verbindungen der Formel (XVI) beispielsweise, indem man substituierte Phenylessigsäuren der Formel (XIX) 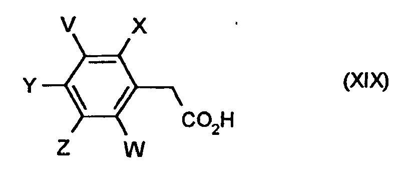 in welcher
- V, W, X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XIX) sind teilweise neu, sie lassen sich nach literaturbekannten Verfahren herstellen (Organikum 15. Auflage, S. 533, VEB Deutscher Verlag der Wissenschaften, Berlin 1977, vgl. z.B.WO 97/02 243, WO 07/01 535 und DE-196 13 171). Man erhält die Verbindungen der Formel (XIX) beispielsweise, indem man substituierte Phenylessigsäureester der Formel (XX) 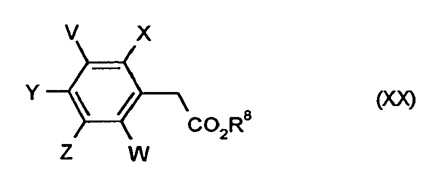 in welcher
- V, W, X, Y, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XX) sind teilweise neu, sie lassen sich nach im Prinzip bekannten Verfahren herstellen.

Man erhält die Verbindungen der Formel (XX) beispielsweise, indem man substituierte 1,1,1-Trichlor-2-phenylethane der Formel (XXI) 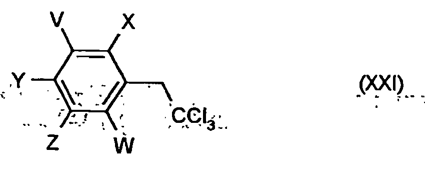 in welcher
- V, W, X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE-3 314 249).

Die Verbindungen der Formel (XXI) sind teilweise neu, sie lassen sich nach im Prinzip bekannten Verfahren herstellen.

Man erhält die Verbindungen der Formel (XXI) beispielsweise, wenn man Aniline der Formel (XXII) 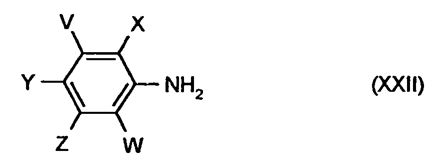 in welcher
- V, W, X, Y und Z: die oben angegebene Bedeutung haben,
in Gegenwart eines Alkylnitrits der Formel (XXIII)

R¹³-ONO (XXIII)

in welcher
- R¹³: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XXII) sind teilweise bekannte Verbindungen. Sie lassen sich aber nach literaturbekannten Verfahren darstellen, beispielsweise durch Reduktion der entsprechenden Nitroverbindungen oder Halogenierung der Aniline bzw. Acetanilide und anschließende Rückspaltung.

Die Verbindungen der Formel (XXIII) sind bekannte Verbindungen der Organischen Chemie. Kupfer(II)-chlorid und Vinylidenchlorid sind lange bekannt und käuflich.

Die Verbindungen der Formel (XV) und (XVIII) sind neu. Sie lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 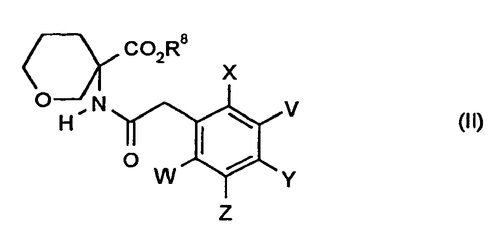 in welcher
- V, W, X, Y, Z und R⁸: die oben angegebenen Bedeutungen haben,
herstellen, wenn man 3-Amino-tetrahydropyran-3-carbonsäurenitril der Formel (XXIV) 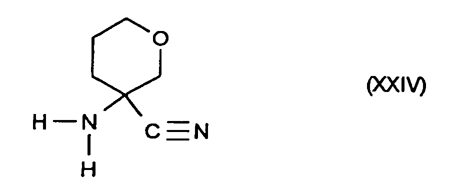 mit substituierten Phenylessigsäurehalogeniden der Formel (XVI) 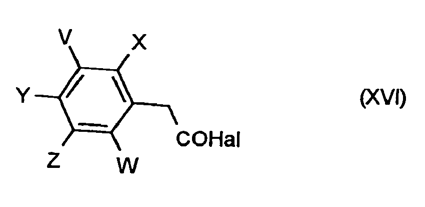 in welcher
- V, W, X, Y, Z und Hal: die oben angegebenen Bedeutungen haben,
zu Verbindungen der Formel (XXV) 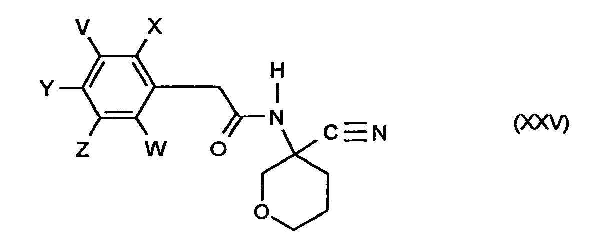 in welcher
- V, W, X, Y und Z: die oben angegebenen Bedeutungen haben,
umsetzt,
und diese anschließend einer sauren Alkoholyse unterwirft.

Die Verbindungen der Formel (XXV) sind ebenfalls neu. Die Verbindung der Formel (XXIV) ist ebenfalls neu (siehe Herstellungsbeispiel).

Die zur Durchführung der erfindungsgemäßen Verfahren (D), (E), (F), (G), (H), (I) und (J) außerdem als Ausgangsstoffe benötigten Säurehalogenide der Formel (V), Carbonsäureanhydride der Formel (VI), Chlorameisensäureester oder Chlorameisensäurethioester der Formel (VII), Chlormonothioameisensäureester oder Chlordithioameisensäureester der Formel (VIII), Sulfonsäurechloride der Formel (IX), Phosphorverbindungen der Formel (X) und Metallhydroxide, Metallalkoxide oder Amine der Formel (XI) und (XII) und Isocyanate der Formel (XIII) und Carbamidsäurechloride der Formel (XIV) sind allgemein bekannte Verbindungen der organischen bzw. anorganischen Chemie.

Die Verbindungen der Formeln (XVI), (XIX), (XX), (XXI) und (XXII) sind darüber hinaus aus den eingangs zitierten Patentanmeldungen bekannt und/oder lassen sich nach den dort angegebenen Methoden herstellen (vgl. auch WO 96/35 664, WO 97/01 535, WO 97/02 243 und DE-196 13 171).

Das Verfahren (A) ist dadurch gekennzeichnet, daß man Verbindungen der Formel (II), in welcher V, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (Dα) ist dadurch gekennzeichnet, daß man Verbindungen der Formel (I-1-a) mit Carbonsäurehalogeniden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Dα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Dα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Dα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Dα) werden die Ausgangsstoffe der Formel (I-1-a) und das Carbonsäurehalogenid der Formel (V) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Dβ) ist dadurch gekennzeichnet, daß man Verbindungen der Formel (I-1-a) mit Carbonsäureanhydriden der Formel (VI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Dβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Dβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Dβ) innerhalb eines größeren Bereiches variiert werden. Im allgemeinen arbeitet man bei Temperaturen zwischen -20°C und + 150°C, vorzugsweise zwischen 0°C und 100°C.

Bei der Durchführung des erfindungsgemäßen Verfahrens (Dβ) werden die Ausgangsstoffe der Formel (I-1-a) und das Carbonsäureanhydrid der Formel (VI) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (E) ist dadurch gekennzeichnet, daß man Verbindungen der Formel (I-1-a) mit Chlorameisensäureestern oder Chlorameisensäurethiolestern der Formel (VII) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (E) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (E) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (E) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (E) wird im allgemeinen unter Normaldruck durchgeführt.

Bei der Durchführung des erfindungsgemäßen Verfahrens (E) werden die Ausgangsstoffe der Formel (I-1-a) und der entsprechende Chlorameisensäureester bzw. Chlorameisensäurethiolester der Formel (VII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (F) ist dadurch gekennzeichnet, daß man Verbindungen der Formel (I-1-a) jeweils mit Verbindungen der Formel (VIII) in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a) ca. 1 Mol Chlormonothioameisensäureester bzw. Chlordithioameisensäureester der Formel (VIII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) dar, kann auf den weiteren Zusatz von Säurebindemitteln verzichtet werden.

Als Basen können beim Verfahren (F) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, daß man Verbindungen der Formel (I-1-a) jeweils mit Sulfonsäurechloriden der Formel (IX) gegebenenfalls in Gegenwart eines Verdünnungsmittels und gegebenenfalls in Gegenwart eines Säurebindemittels umsetzt.

Beim Herstellungsverfahren (G) setzt man pro Mol Ausgangsverbindung der Formel (I-1-a) bis (I-3-a) ca. 1 Mol Sulfonsäurechlorid der Formel (IX) bei -20 bis 150°C, vorzugsweise bei 0 bis 70°C um.

Das Verfahren (G) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (H) ist dadurch gekennzeichnet, daß man Verbindungen der Formel (I-1-a) jeweils mit Phosphorverbindungen der Formel (X) gegebenenfalls in Gegenwart eines Verdünnungsmittels und gegebenenfalls in Gegenwart eines Säurebindemittels umsetzt.

Beim Herstellungsverfahren (H) setzt man zum Erhalt von Verbindungen der Formeln (I-1-e) auf 1 Mol der Verbindungen (I-1-a), 1 bis 2, vorzugsweise 1 bis 1,3 Mol der Phosphorverbindung der Formel (X) bei Temperaturen zwischen -40°C und 150°C, vorzugsweise zwischen -10 und 110°C um.

Das Verfahren (H) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d.h. Entfernung der flüchtigen Bestandteile im Vakuum gereinigt.

Das Verfahren (I) ist dadurch gekennzeichnet, daß man Verbindungen der Formeln (I-1-a) jeweils mit Metallhydroxiden bzw. Metallalkoxiden der Formel (XI) oder Aminen der Formel (XII), gegebenenfalls in Gegenwart eines Verdünnungsmittels, umsetzt.

Als Verdünnungsmittel können bei dem erfindungsgemäßen Verfahren (I) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (I) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (J) ist dadurch gekennzeichnet, daß man Verbindungen der Formeln (I-1-a) jeweils mit (Jα) Verbindungen der Formel (XIII) gegebenenfalls in Gegenwart eines Verdünnungsmittels und gegebenenfalls in Gegenwart eines Katalysators oder (Jβ) mit Verbindungen der Formel (XIV) gegebenenfalls in Gegenwart eines Verdünnungsmittels und gegebenenfalls in Gegenwart eines Säurebindemittels umsetzt.

Bei Herstellungsverfahren (Jα) setzt man pro Mol Ausgangsverbindung der Formeln (I-1-a) ca. 1 Mol Isocyanat der Formel (XIII) bei 0 bis 100°C, vorzugsweise bei 20 bis 50°C um.

Das Verfahren (Jα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Jβ) setzt man pro Mol Ausgangsverbindung der Formeln (I-1-a) ca. 1 Mol Carbamidsäurechlorid der Formel (XIV) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Frankliniella occidentalis, Hercinothrips femoralis, Thrips palmi, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Liriomyza spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.. Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) und gegen die Raupen der Kohlschabe (Plutella maculipennis) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 % und daneben bevorzugt Streckmittel und/oder oberflächenaktive Mittel.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

### Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Di-chloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyano-phenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate, Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol, Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocärb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxyphenoxy-alkansäureester, wie z.B. Diclofop-methyl, Fenoxaprop-ethyl, Fluazifopbutyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, ,Acetochlor, Butachlor, Metazachlor, Metolachlor, Pretilachlor und Propachlor, Dinitroaniline; wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta-sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze wie

Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel-(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octyl-isothiazolin-3-on genannt.

Die Herstellung und die Verwendung der erfindungsgemäßen Wirkstoffe geht aus den nachfolgenden Beispielen hervor.

### Beispiele

### Beispiel I-1-a-1

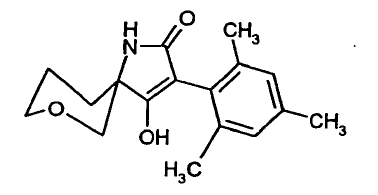

Zu 14,05 g (0,125 mol) Kalium-tert-butylat in 54 ml absolutem Tetrahydrofuran (THF) tropft man bei Rückflußtemperatur 17,3 g der Verbindung gemäß Beispiel II-1 in 160 ml absolutem Toluol und rührt noch 1,5 Stunden bei dieser Temperatur. Nach dem Abkühlen gibt man 160 ml Wasser zu, trennt die Phasen, extrahiert die Toluolphase mit 80 ml Wasser und säuert die vereinigten wässrigen Phasen bei 0 bis 20°C mit konzentrierter HCl an. Man saugt ab, wäscht, trocknet und verrührt das Produkt zur weiteren Reinigung mit Methyl-tert.-butyl-(MTB)-ether/n-Hexan, saugt ab und trocknet. Ausbeute: 12,1 g (77 % der Theorie), Fp.: 200°C.

Analog bzw. gemäß den allgemeinen Angaben zur Herstellung erhält man die folgenden Verbindungen der Formel (I-1-a):

**Tabelle 4**

| | | | | | | |
|---|---|---|---|---|---|---|
| 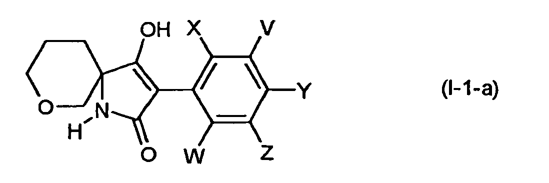 | | | | | | |

| Bsp.-Nr. | V | W | X | Y | Z | Fp. °C |
|---|---|---|---|---|---|---|
| I-1-a-2 | H | CH₃ | CH₃ | CH₃ | CH₃ | 219 |
| I-1-a-3 | H | CH₃ | CH₃ | Br | H | >220 |
| I-1-a-4 | H | H | CH₃ | CH₃ | H | 226 |
| I-1-a-5 | H | H | Cl | Cl | H | >220 |
| I-1-a-6 | H | H | CH₃ | CH₃ | CH₃ | 130 |
| I-1-a-7 | H | H | CH₃ | H | CH₃ | 187 |
| I-1-a-8 | H | Br | Cl | CH₃ | H | >220 |
| I-1-a-9 | H | H | CH₃ | Br | CH₃ | >220 |
| I-1-a-10 | H | Cl | CH₃ | Cl | H | >220. |
| I-1-a-11 | H | H | Br | CH₃ | CH₃ | 145 |
| I-1-a-12 | H | CH₃ | CH₃ | H | Br | 194 |
| I-1-a-13 | H | CH₃ | CH₃ | CH₃ | Br | |

### Beispiel (I-1-b-1)

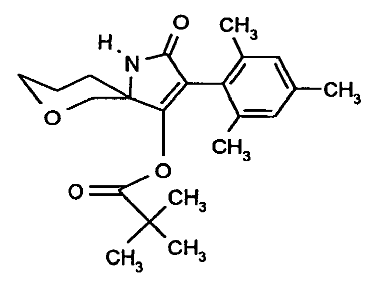

2,88 g (0,010 mol) der Verbindung gemäß Beispiel (I-1-a-1) und 2,1 ml (15 mmol) Triethylamin in 50 ml wasserfreiem Essigsäureethylester werden unter Rückfluß mit 1,85 ml (0,15 mol) Pivaloylchlorid in 5 ml wasserfreiem Essigsäureethylester versetzt. Unter Rückfluß wird gerührt, bis die Reaktion nach dünnschichtchromatographischer Kontrolle beendet ist. Zur Aufarbeitung wird der Ansatz eingeengt, in Methylenchlorid aufgenommene, 2 mal mit 50 ml 0,5 N Natronlauge gewaschen, über Magnesiumsulfat getrocknet und eingedampft. Das Rohprodukt wird aus MTB-Ether/n-Hexan umkristallisiert. Ausbeute: 2,0 g (53 % der Theorie), Fp:: 193 °C.

Analog bzw. gemäß den allgemeinen Angaben zur Herstellung erhält man die folgenden Verbindungen der Formel (I-b-1):

**Tabelle 5**

| | | | | | | | |
|---|---|---|---|---|---|---|---|
| 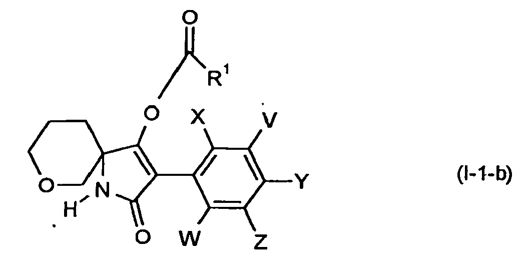 | | | | | | | |

| Bsp.- Nr. | V | W | X | Y | Z | R¹ | Fp. °C |
|---|---|---|---|---|---|---|---|
| I-1-b-2 | H | CH₃ | CH₃ | CH₃ | H | i-C₃H₇ | 163 |
| I-1-b-3 | H | CH₃ | CH₃ | CH₃ | CH₃ | i-C₃H₇ | 153 |
| I-1-b-4 | H | CH₃ | CH₃ | CH₃ | CH₃ | 2-Thienyl | >220 |
| I-1-b-5 | H | CH₃ | CH₃ | Br | H | i-C₃H₇ | 190 |
| I-1-b-6 | H | CH₃ | CH₃ | Br | H | 2-Thienyl | >220 |
| I-1-b-7 | H | CH₃ | CH₃ | Br | H | 4-Cl-Phenyl | 209 |
| I-1-b-8 | H | CH₃ | H | H | CH₃ | i-C₃H₇ | 178 |
| I-1-b-9 | H | CH₃ | H | CH₃ | CH₃ | i-C₃H₇ | 173 |
| I-1-b-10 | H | CH₃ | H | CH₃ | CH₃ | s-C₄H₉ | 193 |
| I-1-b-11 | H | CH₃ | H | CH₃ | H | i-C₃H₇ | 183 |
| I-1-b-12 | H | Cl | H | Cl | H | i-C₃H₇ | 108 |

### Beispiel (I-1-c-1)

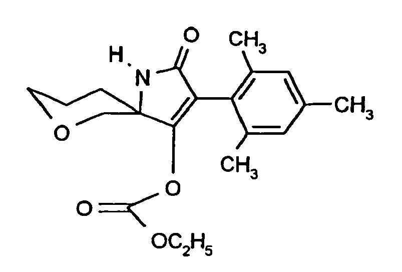

Zu 2,88 g (0,010 mol) der Verbindung gemäß Beispiel (I-1-a-1) und 1,4 ml (0,010 mol) Triethylamin in 50 ml wasserfreiem CH₂Cl₂ tropft man bei 0 bis 10°C 1,0 ml (0,010 mol) Chlorameisensäureethylester in 5 ml wasserfreiem Methylenchlorid und rührt bei Raumtemperatur, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft. Ausbeute: 2,8 g (77 % der Theorie), Fp.: 157°C.

Analog bzw. gemäß den allgemeinen Angaben zur Herstellung erhält man die folgenden Verbindungen der Formel (I-1-c):

**Tabelle 6**

| | | | | | | | | | |
|---|---|---|---|---|---|---|---|---|---|
| 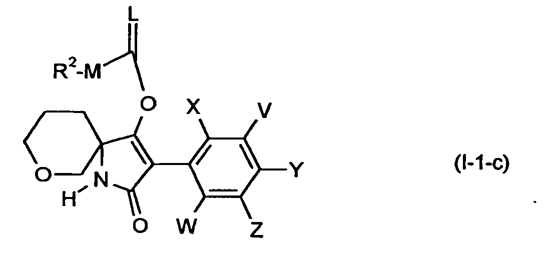 | | | | | | | | | |

| Bsp.- Nr. | V | W | X | Y | Z | L | M | R² | Fp. °C |
|---|---|---|---|---|---|---|---|---|---|
| I-1-c-2 | H | CH₃ | CH₃ | CH₃ | CH₃ | O | O | C₂H₅ | 163 |
| I-1-c-3 | H | CH₃ | CH₃ | Br | H | O | O | C₂H₅ | 184 |
| I-1-c-4 | H | H | Cl | Cl | H | O | O | C₂H₅ | 131 |

**Beispiel II-1**

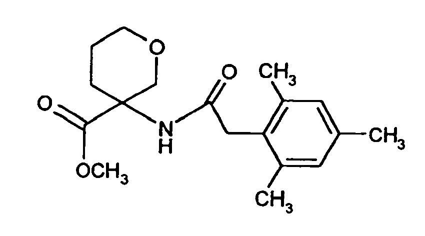

Zu 51,3 g (0,513 mol) konz. Schwefelsäure tropft man bei 30 bis 40°C vorsichtig 29,1 g (0,1016 mol) der Verbindung gemäß Beispiel (XXV-1) in 310 ml wasserfreiem Methylenchlorid und rührt 2 Stunden bei dieser Temperatur. Dann tropft man 69 ml absolutes Methanol so zu, daß sich eine Innentemperatur von etwa 40°C einstellt und rührt noch 6 Stunden bei 40 bis 70°C.

Zur Aufarbeitung gießt man auf 0,51 kg Eis, extrahiert mit Methylenchlorid, wäscht mit wäßriger Natriumhydrogencarbonatlösung, trocknet und dampft ein. Der Rückstand wird aus Methyl-tert.-butylether/n-Hexan umkristallisiert. Ausbeute: 17,3 g (53 % der Theorie), Fp.: 168°C.

Analog zu Beispiel (II-1) und gemäß den allgemeinen Angaben zur Herstellung werden die folgenden Verbindungen der Formel (II) hergestellt.

**Tabelle 7**

| | | | | | | | |
|---|---|---|---|---|---|---|---|
| 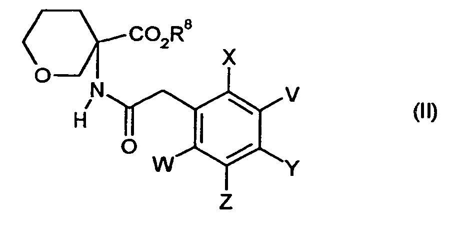 | | | | | | | |

| Bsp.-Nr. | V | W | X | Y | Z | R⁸ | Fp. °C |
|---|---|---|---|---|---|---|---|
| II-2 | H | CH₃ | CH₃ | CH₃ | CH₃ | CH₃ | 78 |
| II-3 | H | CH₃ | CH₃ | Br | H | CH₃ | 110 |
| II-4 | H | H | CH₃ | CH₃ | H | CH₃ | 68 |
| II-5 | H | H | Cl | Cl | H | CH₃ | 103 |
| II-6 | H | H | CH₃ | CH₃ | CH₃ | CH₃ | 92 |
| II-7 | H | Br | Cl | CH₃ | H | CH₃ | 114 |
| II-8 | H | Cl | CH₃ | Cl | H | CH₃ | 204 |
| II-9 | H | H | CH₃ | H | CH₃ | CH₃ | 74 |
| II-10 | H | H | CH₃ | H | H | CH₃ | 85 |
| II-11 | H | H | Br | CH₃ | CH₃ | CH₃ | 81 |
| II-12 | H | H | CH₃ | Br | CH₃ | CH₃ | 110 |
| II-13 | H | CH₃ | CH₃ | H | Br | CH₃ | 134 |
| II-14 | H | H | Cl | | -O-CF₂-O- | CH₃ | 121 |
| II-15 | H | CH₃ | CH₃ | CH₃ | Br | CH₃ | 97 |

### Beispiel XXV-1

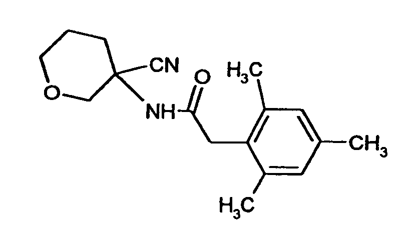

Zu 15,8 g 3-Aminotetrahydropyran-3-carbonsäurenitril (gemäß Beispiel XXTV) und 17,6 ml Triethylamin in 250 ml absolutem THF tropft man bei 0 bis 10°C 25 g Mesitylenessigsäurechlorid in 30 ml absolutem THF und rührt, bis die Reaktion beendet ist. Die Mischung wird in ein Gemisch von 500 ml Eiswasser und 200 ml 1 N HCl eingerührt. Man saugt ab, nimmt den Rückstand in Methylenchlorid auf, trocknet und engt ein. Ausbeute 29,1 g (81 % der Theorie), Fp. 153°C.

Analog zu Beispiel (XXV-1) bzw. gemäß den allgemeinen Angaben zur Herstellung werden folgende Verbindungen der Formel (XXV) hergestellt:

**Tabelle 8**

| | | | | | | |
|---|---|---|---|---|---|---|
| 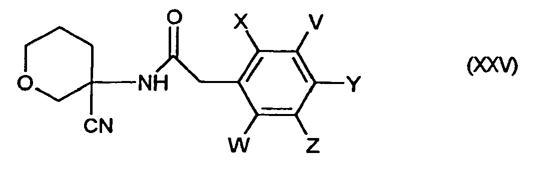 | | | | | | |

| Bsp.-Nr. | V | W | X | Y | Z | Fp.°C |
|---|---|---|---|---|---|---|
| XXV-2 | H | CH₃ | CH₃ | CH₃ | CH₃ | 182 |
| XXV-3 | H | CH₃ | CH₃ | Br | H | 187 |
| XXV-4 | H | H | CH₃ | CH₃ | H | 137 |
| XXV-5 | H | H | Cl | Cl | H | 162 |
| XXV-6 | H | H | CH₃ | CH₃ | CH₃ | 159 |
| XXV-7 | H | Br | Cl | CH₃ | H | 158 |
| XXV-8 | H | Cl | CH₃ | Cl | H | 141 |
| XXV-9 | H | H | CH₃ | H | CH₃ | 148 |
| XXV-10 | H | H | CH₃ | H | H | 149 |
| XXV-11 | H | CH₃ | CH₃ | H | Br | 176 |
| XXV-12 | H | H | Br | CH₃ | CH₃ | 135 |
| XXV-13 | H | H | CH₃ | Br | CH₃ | 166 |
| XXV-15 | H | CH₃ | CH₃ | CH₃ | Br | 212 |

### Beispiel XXIV

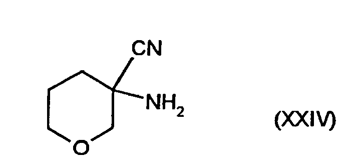

Zum Gemisch aus 222,4 g (3,27 mol) 25%iger Ammoniaklösung, 75 g (1,4 mol) Ammoniumchlorid, 68,7 g (1,4 mol) Natriumcyanid und 210 ml Wasser tropft man bei Raumtemperatur 116,8 g (1,17 mol) 3-Oxa-cyclohexanon-1 (siehe Bull. Soc. Chim. Fr. (1970), (10), 3521-3) und rührt über Nacht bei 45°C. Nach Extraktion mit Methylenchlorid erhielt man 95,2 g (64 % der Theorie), der oben gezeigten Verbindung, die ohne weitere Aufreinigung in die Herstellung der Verbindungen der Formel XXV eingesetzt wurde.

### Anwendungsbeispiele

### Beispiel A

### Myzus-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit werden die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen (I-1-a-2), (I-1-a-3), (I-1-b-2) und (I-1-c-1) bei einer beispielhaften Wirkstoffkonzentration von 0,1 % eine Abtötung von 100 % nach 6 Tagen.

### Beispiel B

### Nephotettix-Test

- Lösungsmittel:: 20 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen (I-1-a-1), (I-1-a-2), (I-1-a-3), (I-1-b-1), (I-1-b-2), (I-1-b-3), (I-1-b-4), (I-1-b-5) und (I-1-c-3) bei einer beispielhaften Wirkstoffkonzentration von 0,1 % eine Abtötung von 100 % nach 6 Tagen.

### Beispiel C

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test bewirkten z.B. die Verbindungen gemäß den Herstellungsbeispielen (I-1-a-1), (I-1-a-2), (I-1-a-3), (I-1-b-1), (I-1-b-2), (I-1-b-3), (I-1-b-4), (I-1-b-5) und (I-1-c-1) bei einer beispielhaften Wirkstoffkonzentration von 0,1 % eine Abtötung von 100 % nach 7 Tagen.

### Beispiel D

### Spodoptera frugiperda-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen (I-1-b-4) und (I-1-c-1) bei einer beispielhaften Wirkstoffkonzentration von 0,1 % eine Abtötung von 100 % nach 7 Tagen.

### Beispiel E

### Tetranychus-Test (OP-resistent/Tauchbehandlung)

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Stadien der gemeinen Spinnmilbe Tetranychus urticae befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test bewirkten z.B. die Verbindungen gemäß den Herstellungsbeispielen (I-1-a-1), (I-1-b-1), (I-1-b-2) und (I-1-c-1) bei einer beispielhaften Wirkstoffkonzentration von 0,1 % eine Abtötung von 100 % nach 13 Tagen.

## Patentansprüche

1. Verbindungen der Formel (I) 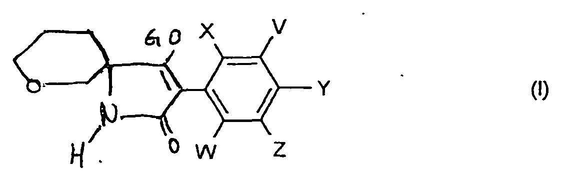 in welcher
V für Wasserstoff, Halogen, C₁-C₆-Alkyl oder C₁-C₆-Alkoxy steht,
W für Wasserstoff, Nitro, Cyano, Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy oder für jeweils gegebenenfalls durch Halogen, C₁-C₆-Alkyl, C₁-C₆-Alkoxy, C₁-C₄-Halogenakyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht,
X für Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy, Cyano, Nitro oder für jeweils gegebenenfalls durch Halogen, C₁-C₆-Alkyl, C₁-C₆-Alkoxy, C₁-C₄-Halogenakyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio steht,
Y für Wasserstoff, Halogen, C₁-C₆-Alkyl, C₁-C₆-Alkoxy, C₁-C₄-Halogenalkyl, C₁-C₄-Halogenalkoxy, Cyano oder Nitro steht,
Z für Wasserstoff, Halogen, C₁-C₆-Alkyl, C₁-C₆-Alkoxy, C₁-C₄-Halogenalkyl, C₁-C₄-Halogenalkoxy, Hydroxy, Cyano, Nitro oder für jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht oder
steht,
G für Wasserstoff (a) oder für eine der Gruppen 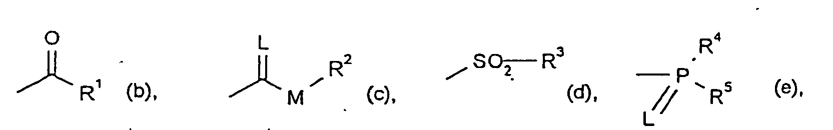 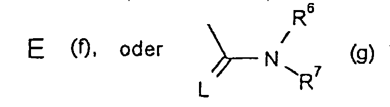 steht,
in welchen
E für ein Metallionäquivalent oder ein Ammoniumion steht,
L für Sauerstoff oder Schwefel steht und
M für Sauerstoff oder Schwefel steht.
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alky, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl steht, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alköxy, C₁-C₆-Halogenalkyl, C₁-C₆-Halogenallcoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₅-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl C₁-C₆-Alkoxy, C₁-C₄-Halogenallcyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
R⁶ und R⁷ unabhängig voneinander für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

2. Verbindungen der Formel (I) gemäß Anspruch 1, in welcher
V für Wasserstoff, Fluor, Chlor, Brom, C₁-C₄-Alkyl oder C₁-C₄-Alkoxy steht,
W für Wasserstoff, Nitro, Cyano, Fluor, Chlor, Brom, C₁-C₄-Alkyl, C₁-C₄-Alkoxy, C₁-C₂-Halogenalkyl, C₁-C₂-Halogenalkoxy oder für jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy oder Benzyloxy steht,
X für Fluor, Chlor, Brom, C₁-C₄-Alkyl, C₁-C₄-Alkoxy, C₁-C₂-Halogenalkyl, C₁-C₂-Halogenalkoxy, Cyano, Nitro oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy oder Benzyloxy steht,
Y für Wasserstoff; Fluor, Chlor, Brom, C₁-C₄-Alkyl, C₁-C₄-Alkoxy, C₁-C₂-Halogenalkyl, C₁-C₂-Halogenalkoxy, Cyano oder Nitro steht,
Z für Wasserstoff: Fluor, Chlor, Brom, C₁-C₄-Alkyl, C₁-C₄-Alkoxy, C₁-C₂ Halogenalkyl, C₁-C₂-Halogenalkoxy, Hydroxy, Cyano, Nitro oder für jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenoxy oder Benzyloxy steht oder steht,
G für Wasserstoff (a) oder für eine der Gruppen 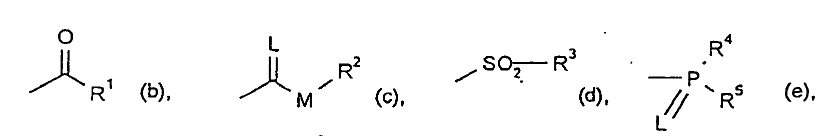 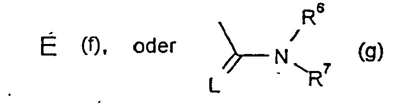 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalky, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht,
R³ für gegebenenfalls durch Fluor oder Chlor substituiertes C₁-C₆-Alkyl oder für jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls durch Fluor oder Chlor substituiertes C₁-C₆-Alkyl, C₁-C₆-Akoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen und
R⁶ und R⁷ unabhängig voneinander für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist.

3. Verbindungen der Formel (I) gemäß, Anspruch 1, in welcher
V für Wasserstoff, Fluor, Chlor, Brom, Methyl, Ethyl, Propyl, iso-Propyl, tert-Butyl, Methoxy, Ethoxy, Propoxy oder iso-Propoxy steht,
W für Wasserstoff, Nitro, Cyano, Fluor, Chlor, Brom, Methyl, Ethyl, Propyl, n-Butyl, iso-Propyl, iso-Butyl, Methoxy, Ethoxy, Propoxy, iso-Propoxy, Trifluormethyl, Difluormethoxy, Trifluormethoxy, Phenyl oder Benzyloxy steht
X für Fluor, Chlor, Brom, Methyl, Ethyl, Propyl, Butyl, iso-Butyl, iso-Propyl, Methoxy, Ethoxy, Propoxy, iso-Propoxy, Trifluormethyl, Trifluormethoxy, Difluormethoxy, Cyano, Nitro, Phenyl oder Benzyloxy steht,
Y für Wasserstoff, Fluor, Chlor, Brom, Methyl, Ethyl, Propyl, iso-Propyl, n-Butyl, iso-Butyl, tert.-Butyl, Methoxy, Ethoxy, Propoxy, iso-Propoxy, Trifluormethyl, Trifluormethoxy, Difluormethoxy, Cyano oder Nitro steht,
Z für Wasserstoff Fluor, Chlor, Brom, Methyl, Ethyl, Propyl, Butyl, iso-Butyl, iso-Propyl, tert.-Butyl; Methoxy, Ethoxy, Propoxy, iso-Propoxy, Trifluormethyl, Trifluormethoxy, Difluormethoxy, Cyano oder Nitro steht oder steht,
G für Wasserstoff (a) oder für eine der Gruppen 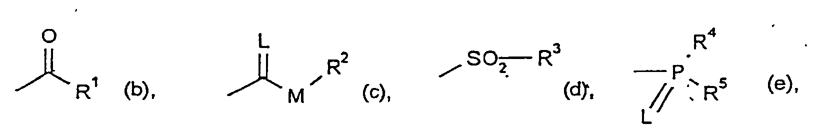 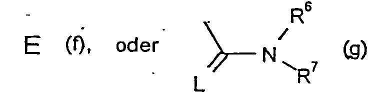 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoaxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyäno, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder '
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl steht,
R² für jeweils gegebenenfalls durch Fluor oder Chlor substituiertes C₁-C₁₄-Alky, C₂-C₁₄-Alkeny, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für jeweils gegebenenfalls durch Fluor oder Chlor substituiertes Methyl, Ethyl, Propyl, iso-Propyl, n-Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen und
R⁶ und R⁷ unabhängig voneinander für Wasserstoff, für jeweils gegebenenfalls durch Fluor oder Chlor substituiertes C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest stehen, in welchem gegebenenfalls eine Methylengruppe durch Sauerstoff oder Schwefel ersetzt ist.

4. Verfahren zur Herstellung von Verbindungen der Formel (I) gemäß Anspruch 1-3, **dadurch gekennzeichnet, daß** man
(A) Verbindungen der Formel (I-1-a) 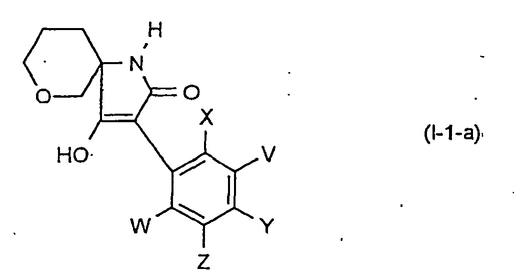 in welcher
V, W, X, Y und Z die in Anspruch 1 angegebenen Bedeutungen haben, erhält,
wenn man
Verbindungen der Formel (II) 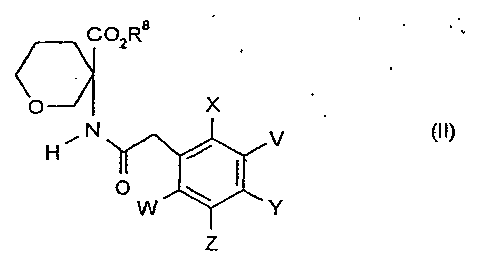 in welcher
V, W, X, Y und Z die oben angegebenen Bedeutungen haben,
und
R⁸ für Alkyl steht,
in Gegenwart eines Verdünnungsmittels und in Gegenwart einer Base intramolekular kondensiert,
und anschließend gegebenenfalls die so erhaltenen Verbindungen der oben gezeigten Formeln (I-1-a), in welchen V, W, X, Y und Z die oben angegebenen Bedeutungen haben, jeweils
(D) α) mit Säurehalogeniden der Formel (V) 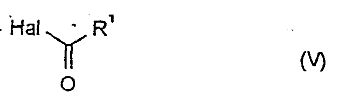 in welcher
R¹ die in Anspruch 1 angegebene Bedeutung hat und
Hal für Halogen steht
oder
β) mit Carbonsäureanhydriden der Formel (VI)
R¹-CO-O-CO-R¹ (VI)
in welcher
R¹ die oben angegebene Bedeutung hat,
gegebenenfalls in Gegenwart eines Verdünnungsmittels und gegebenenfalls in Gegenwart eines Säurebindemittels umsetzt; oder
(E) mit Chlorameisensäureestern oder Chlorameisensäurethioestern der Formel (VII)
R²-M-CO-Cl (VII)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(F) mit Chlormonothioameisensäureestern oder Chlordithioameisensäureestern der Formel (VIII) 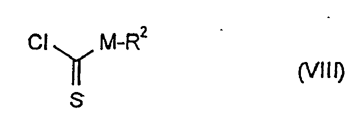 in welcher
M und R² die in Anspruch 1 angegebenen Bedeutungen haben,
gegebenenfalls in Gegenwart eines Verdünnungsmittels und gegebenenfalls in Gegenwart eines Säurebindemittels umsetzt,
oder
(G) mit Sulfonsäurechloriden der Formel (IX)
R³-SO₂-Cl (IX)
in welcher
R³ die in Anspruch 1 angegebene Bedeutung hat,
gegebenenfalls in Gegenwart eines Verdünnungsmittels und gegebenenfalls in Gegenwart eines Säurebindemittels umsetzt; oder
(H) mit Phosphorverbindungen der Formel (X) 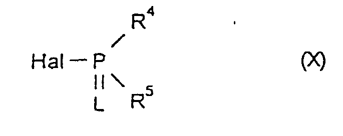 in welcher
L, R⁴ und R⁵ die in Anspruch 1 angegebenen Bedeutungen haben und
Hal für Halogen steht,
gegebenenfalls in Gegenwart eines Verdünnungsmittels und gegebenenfalls in Gegenwart eines Säurebindemittels umsetzt; oder
(I) mit Metallverbindungen oder Aminen der Formeln (XI) oder (XII)
Me(OR¹⁰)ₜ (XI) 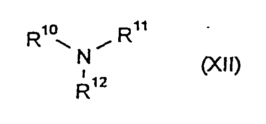
in welchen
Me für ein ein- oder zweiwertiges Metall
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
oder
(J)
α) mit Isocyanaten oder Isothiocyanaten der Formel (XIII)
R⁶-N=C=L (XIII)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt oder
β) mit Carbamidsäurechloriden oder Thiocarbamidsäurechloriden der Formel (XIV) 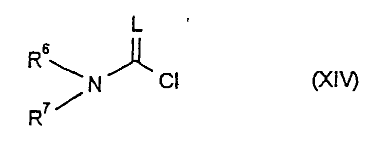 in welcher
L, R⁶ und R⁷ die in Anspruch 1 angegebenen Bedeutungen haben,
gegebenenfalls in Gegenwart eines Verdünnungsmittels und gegebenenfalls in Gegenwart eines Säurebindemittels, umsetzt.

5. Verbindungen der Formel (II) 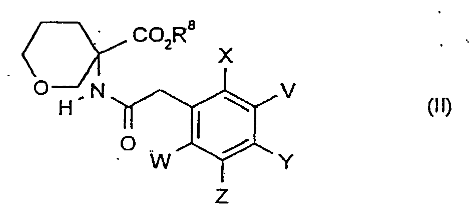 in welcher
V, W, X, Y und Z die in Anspruch 1 angegebenen Bedeutungen haben und
R⁸ für Alkyl steht.

6. Verbindungen der Formel (XVII) 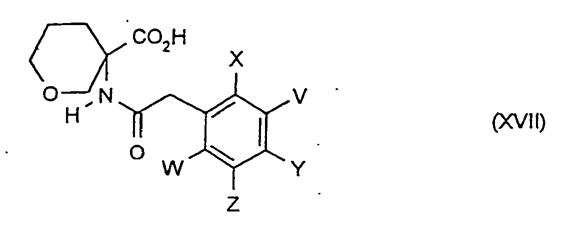 in welcher
V, W, X, Y und Z die in Anspruch 1 angegebenen Bedeutungen haben.

7. Verbindungen der Formel (XXV) 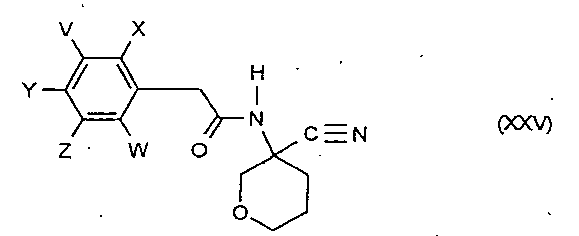 in welcher
V, W, X, Y und Z die in Anspruch 1 angegebenen Bedeutungen haben.

8. Verbindung der Formel (XXIV)

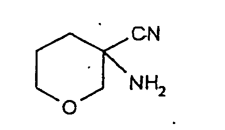

9. Verbindungen der Formel 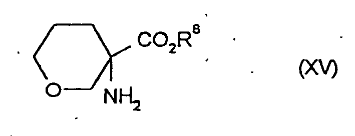 in welcher
R⁸ für Alkyl steht.

10. Verbindung der Formel

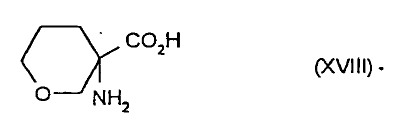

11. Schädlingsbekämpfungsmittel oder herbizide Mittel, **gekennzeichnet durch** einen Gehalt an einer Verbindung der Formel (I) gemäß Anspruch 1-3.

12. Verwendung von Verbindungen der Formel (I) gemäß Anspruch 1-3 zur Bekämpfung von Schädlingen und Unkräutern, ausgenommen aus menschlichen oder tierischen Körper.

13. Verfahren zur Bekämpfung von Schädlingen und Unkräutern, **dadurch gekennzeichnet, daß** man Verbindungen der Formel (I) gemäß Anspruch 1-3 auf Schädlinge bzw. Unkräuter und/oder ihren Lebensraum einwirken läßt, ausgenommen am menschlichen oder tierischen Körper.

14. Verfahren zur Herstellung von Schädlingsbekämpfungsmitteln und herbiziden Mitteln, **dadurch gekennzeichnet, daß** man Verbindungen der Formel (I) gemäß Anspruch 1-3 mit Streckmitteln und/oder oberflächenaktiven Mitteln vermischt.

## Claims

1. Compounds of the formula (I) 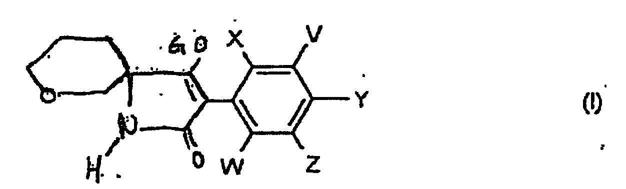 in which
V represents hydrogen, halogen, C₁-C₆-alkyl or C₁-C₆-alkoxy,
W represents hydrogen, nitro, cyano, halogen, C₁-C₆-alkyl, C₂-C₆-alkenyl, C₂-C₆-alkinyl, C₁-C₆-alkoxy, C₁-C₄-halogenoalkyl, C₁-C₄-halogenoalkoxy or represents in each case optionally halogen-, C₁-C₆-alkyl-, C₁-C₆-alkoxy-, C₁-C₄-halogenoalkyl-, C₁-C₄-halogenoalkoxy-, nitro- or cyano-substituted phenyl, phenoxy, phenylthio, phenyl-C₁-C₄-alkoxy or phenyl-C₁-C₄-alkylthio,
X represents halogen, C₁-C₆-alkyl, C₂-C₆-alkenyl, C₂-C₆-alkinyl, C₁-C₆-alkoxy, C₁-C₄-halogenoalkyl, C₁-C₄-halogenoalkoxy, cyano, nitro or represents in each case optionally halogen-, C₁-C₆-alkyl, C₁-C₆-alkoxy, C₁-C₄-halogenoalkyl, C₁-C₄-halogenoalkoxy, nitro- or cyano-substituted phenyl, phenoxy, phenylthio, phenyl-C₁-C₄-alkoxy or phenyl-C₁-C₄-alkylthio,
Y represents hydrogen, halogen, C₁-C₆-alkyl, C₁-C₆-alkoxy, C₁-C₄-halogenoalkyl, C₁-C₄-halogenoalkoxy, cyano or nitro,
Z represents hydrogen, halogen, C₁-C₆-alkyl, C₁-C₆-alkoxy, C₁-C₄-halogenoalkyl, C₁-C₄-halogenoalkoxy, hydroxyl, cyano, nitro or represents in each case optionally halogen-, C₁-C₄-alkyl-, C₁-C₄-alkoxy-, C₁-C₄-halogenoalkyl-, C₁-C₄-halogenoalkoxy-, nitro- or cyano-substituted phenoxy, phenylthio, thiazolyloxy, pyridinyloxy, pyrimidyloxy, pyrazolyloxy, phenyl-C₁-C₄-alkyloxy or phenyl-C₁-C₄-alkylthio or
G represents hydrogen (a) or represents one of the groups 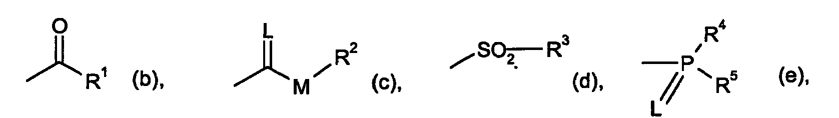 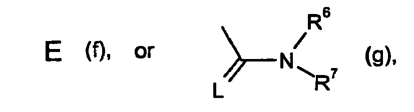 in which
E represents a metal ion equivalent or an ammonium ion,
L represents oxygen or sulphur and
M repersents oxygen or sulphur.
R¹ represents in each case optionally halogen- or cyano-substituted C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl or represents optionally halogen-, C₁-C₆-alkyl- or C₁-C₆-alkoxy-substituted C₃-C₈-cycloalkyl in which optionally one or two not directly adjacent methylene groups are replaced by oxygen and/or sulphur,
represents optionally halogen-, cyano-, nitro-, C₁-C₆-alkyl-, C₁-C₆-alkoxy-, C₁-C₆-halogenoalkyl-, C₁-C₆-halogenoalkoxy-, C₁-C₆-alkylthio- or C₁-C₆-alkylsulfonyl-substituted phenyl,
represents optionally halogen-, nitro-, cyano-, C₁-C₆-alkyl-, C₁-C₆-alkoxy-, C₁-C₆-halogenoalkyl- or C₁-C₆-halogenoalkoxy-substituted phenyl-C₁-C₆-alkyl,
represents optionally halogen- or C₁-C₆-alkyl-substituted 5- or 6-membered hetaryl having one or two heteroatoms from the group consisting of oxygen, sulphur and nitrogen,
represents optionally halogen- or C₁-C₆-alkyl-substituted phenoxy-C₁-C₆-alkyl or
represents optionally halogen-, amino- or C₁-C₆-alkyl-substituted 5- or 6-membered hetaryloxy-C₁-C₆-alkyl having one or two heteroatoms from the group consisting of oxygen, sulphur and nitrogen,
R² represents in each case optionally halogen- or cyano-substituted C₁-C₂₀-alkyl, C₂-C₂₀-alkenyl, C₁-C₈-alkoxy-C₂-C₈-alkyl or poly-C₁-C₈-alkoxy-C₂-C₈-alkyl,
represents optionally halogen-, C₁-C₆-alkyl- or C₁-C₆-akoxy-substituted C₃-C₈-cycloalkyl or
represents in each case optionally halogen-, cyano-, nitro-, C₁-C₆-alkyl-, C₁-C₆-alkoxy-, C₁-C₆-halogenoalkyl- or C₁-C₆-halogenoalkoxy-substituted phenyl or benzyl,
R³ represents optionally halogen-substituted C₁-C₈-alkyl or represents in each case optionally halogen-, C₁-C₆-alkyl-, C₁-C₆-alkoxy-, C₁-C₄-halogenoalkyl-, C₁-C₄-halogenoalkoxy-, cyano- or nitro-substituted phenyl or benzyl,
R⁴ and R⁵ independently of one another each represent in each case optionally halogen-substituted C₁-C₈-alkyl, C₁-C₈-alkoxy, C₁-C₈-alkylamino, di-(C₁-C₈-alkyl)amino, C₁-C₈-alkylthio or C₃-C₈-alkenylthio or represent in each case optionally halogen-, nitro-, cyano-, C₁-C₄-alkoxy-, C₁-C₄-halogenoalkoxy-, C₁-C₄-alkylthio-, C₁-C₄-halogenoalkylthio-, C₁-C₄-alkyl- or C₁-C₄-halogenoalkyl-substituted phenyl, phenoxy or phenylthio and
R⁶ and R⁷ independently of one another each represent hydrogen, represent in each case optionally halogen- or cyano-substituted C₁-C₈-alkyl, C₃-Cg-cycloalkyl, C₁-C₈-alkoxy, C₃-C₈-alkenyl or C₁-C₈-alkoxy-C₂-C₈-alkyl, represent in each case optionally halogen-, C₁-C₈-alkyl-, C₁-C₈-halogenoalkyl- or C₁-C₈-alkoxy-substituted phenyl or benzyl or together represent an optionally C₁-C₆-alkyl-substituted C₃-C₆-alkylene radical in which optionally one methylene group is replaced by oxygen or sulphur.

2. Compounds of the formula (I) according to Claim 1, in which
V represents hydrogen, fluorine, chlorine, bromine, C₁-C₄-alkyl or C₁-C₄-alkoxy,
W represents hydrogen, nitro, cyano, fluorine, chlorine, bromine, C₁-C₄-alkyl, C₁-C₄-alkoxy, C₁-C₂-halogenoalkyl, C₁-C₂-halogenoalkoxy or represents in each case optionally fluorine-, chlorine-, bromine-, C₁-C₄-alkyl-, C₁-C₄-alkoxy-, C₁-C₂-halogenoalkyl-, C₁-C₂-halogenoalkoxy-, nitro- or cyano-substituted phenyl, phenoxy or benzyloxy,
X represents fluorine, chlorine, bromine, C₁-C₄-alkyl, C₁-C₄-alkoxy, C₁-C₂-halogenoalkyl, C₁-C₂-halogenoalkoxy, cyano, nitro or represents in each case optionally fluorine-, chlorine-, bromine-, C₁-C₄-alkyl-, C₁-C₄-alkoxy-, C₁-C₂-halogenoalkyl-, C₁-C₂-halogenoalkoxy-, nitro- or cyano-substituted phenyl, phenoxy or benzyloxy,
Y represents hydrogen, fluorine, chlorine, bromine, C₁-C₄-alkyl, C₁-C₄-alkoxy, C₁-C₂-halogenoalkyl, C₁-C₂-halogenoalkoxy, cyano or nitro,
Z represents hydrogen, fluorine, chlorine, bromine, C₁-C₄-alkyl-, C₁-C₄-alkoxy-, C₁-C₂-halogenoalkyl, C₁-C₂-halogenoalkoxy, hydroxyl, cyano, nitro or represents in each case optionally fluorine-, chlorine-, bromine-, C₁-C₄-alkyl-, C₁-C₄-alkoxy-, C₁-C₂-halogenoalkyl-, C₁-C₂-halogenoalkoxy-, nitro- or cyano-substituted phenoxy or benzyloxy or
G represents hydrogen (a) or represents one of the groups 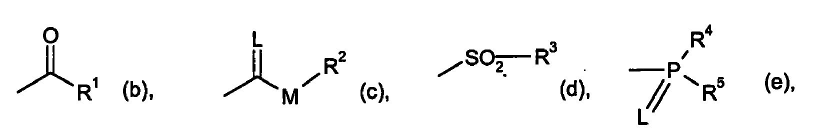 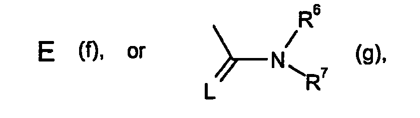 in which
E represents a metal ion equivalent or an ammonium ion,
L represents oxygen or sulphur and
M represents oxygen or sulphur,
R¹ represents in each case optionally fluorine- or chlorine-substituted C₁-C₁₆-alkyl, C₂-C₁₆-alkenyl, C₁-C₆-alkoxy-C₁-C₆-alkyl, C₁-C₆-alkylthio-C₁-C₆-alkyl or poly-C₁-C₆-alkoxy-C₁-C₆-akyl or represents optionally fluorine-, chlorine-, C₁-C₅-alkyl- or C₁-C₅-alkoxy-substituted C₃-C₇-cycloalkyl in which optionally one or two not directly adjacent methylene groups are replaced by oxgyen and/or sulphur,
represents optionally fluorine-, chlorine-, bromine-, cyano-, nitro-, C₁-C₄-alkyl-, C₁-C₄-alkoxy-, C₁-C₃-halogenoalkyl-, C₁-C₃-halogenoalkoxy-, C₁-C₄-alkylthio- or C₁-C₄-alkylsulphonyl-substituted phenyl,
represents optionally fluorine-, chlorine-, bromine-, C₁-C₄-alkyl-, C₁-C₄-alkoxy-, C₁-C₃-halogenoalkyl- or C₁-C₃-halogenoalkoxy-substituted phenyl-C₁-C₄-alkyl,
represents in each case optionally fluorine-, chlorine-, bromine- or C₁-C₄-alkyl-substituted pyrazolyl, thiazolyl, pyridyl, pyrimidyl, furanyl or thienyl,
represents optionally fluorine-, chlorine-, bromine- or C₁-C₄-alkyl-substituted phenoxy-C₁-C₅-alkyl or
represents in each case optionally fluorine-, chlorine-, bromine-amino- or C₁-C₄-alkyl-substituted pyridyloxy-C₁-C₅-alkyl, pyrimidyloxy-C₁-C₅-alkyl or thiazolyloxy-C₁-C₅-alkyl,
R² represents in each case optionally fluorine- or chlorine-substituted C₁-C₁₆-alkyl, C₂-C₁₆-alkenyl, C₁-C₆-alkoxy-C₂-C₆-alkyl or poly-C₁-C₆-alkoxy-C₂-C₆-alkyl,
represents optionally fluorine-, chlorine-, C₁-C₄-akyl- or C₁-C₄-alkoxy-substituted C₃-C₇-cycloalkyl or
represents in each case optionally fluorine-, chlorine-, bromine-, cyano-, nitro-, C₁-C₄-alkyl-, C₁-C₃-alkoxy-, C₁-C₃-halogenoalkyl- or C₁-C₃-halogenoalkoxy-substituted phenyl or benzyl,
R³ represents optionally fluorine- or chlorine-substituted C₁-C₆-alkyl or represents in each case optionally fluorine-, chlorine-, bromine-, C₁-C₄-alkyl-, C₁-C₄-alkoxy-, C₁-C₂-halogenoalkoxy-, C₁-C₂-halogenoalkyl-, cyano- or nitro-substituted phenyl or benzyl,
R⁴ and R⁵ independently of one another each represent in each case optionally fluorine- or chlorine-substituted C₁-C₆-alkyl-, C₁-C₆-alkoxy-, C₁-C₆-alkylamino-, di-(C₁-C₆-alkyl)amino, C₁-C₆-alkylthio or C₃-C₄-alkenylthio or represent in each case optionally fluorine-, chlorine-, bromine-, nitro-, cyano-, C₁-C₃-alkoxy-, C₁-C₃-halogenoalkoxy-, C₁-C₃-alkylthio-, C₁-C₃-halogenoalkylthio-, C₁-C₃-alkyl or C₁-C₃-halogenoalkyl-substituted phenyl, phenoxy or phenylthio and
R⁶ and R⁷ independently of one another each represent hydrogen, represent in each case optionally fluorine- or chlorine-substituted C₁-C₆-alkyl, C₃-C₆-cycloalkyl, C₁-C₆-alkoxy, C₃-C₆-alkenyl or C₁-C₆-alkoxy-C₂-C₆-alkyl, represent in each case optionally fluorine-, chlorine-, bromine-, C₁-C₅-halogenoalkyl-, C₁-C₅-alkyl- or C₁-C₅-alkoxy-substituted phenyl or benzyl, or together represent an optionally C₁-C₄-alkyl-substituted C₃-C₆-alkylene radical in which optionally one methylene group is replaced by oxygen or sulphur.

3. Compounds of the formula (I) according to Claim 1, in which
V represents hydrogen, fluorine, chlorine, bromine, methyl, ethyl, propyl, iso-propyl, tert-butyl, methoxy, ethoxy, propoxy or iso-propoxy,
W represents hydrogen, nitro, cyano, fluorine, chlorine, bromine, methyl, ethyl, propyl, n-butyl, iso-propyl, iso-butyl, methoxy, ethoxy, propoxy, iso-propoxy, trifluoromethyl, difluoromethoxy, trifluoromethoxy, phenyl or benzyloxy
X represents fluorine, chlorine, bromine, methyl, ethyl, propyl, butyl, iso-butyl, iso-propyl, methoxy, ethoxy, propoxy, iso-propoxy, trifluoromethyl, trifluoromethoxy, difluoromethoxy, cyano, nitro, phenyl or benzyloxy,
Y represents hydrogen, fluorine, chlorine, bromine, methyl, ethyl, propyl, iso-propyl, n-butyl, iso-butyl, tert-butyl, methoxy, ethoxy, propoxy, iso-propoxy, trifluoromethyl, trifluoromethoxy, difluoromethoxy, cyano or nitro,
Z represents hydrogen, fluorine, chlorine, bromine, methyl, ethyl, propyl, butyl, iso-butyl, iso-propyl, tert-butyl, methoxy, ethoxy, propoxy, iso-propoxy, trifluoromethyl, trifluoromethoxy, difluoromethoxy, cyano or nitro or
G represents hydrogen (a) or represents one of the groups 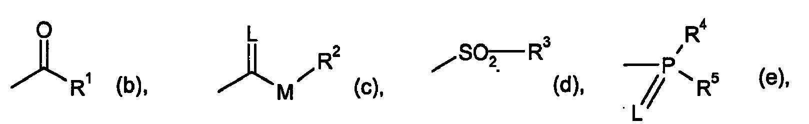 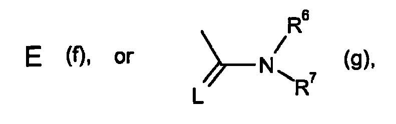 in which
E represents a metal ion equivalent or an ammonium ion,
L represents oxygen or sulphur and
M represents oxygen or sulphur,
R¹ represents in each case optionally chlorine- or fluorine-substituted C₁-C₁₄-alkyl, C₂-C₁₄-alkenyl, C₁-C₄-alkoxy-C₁-C₆-alkyl, C₁-C₄-alkylthio-C₁-C₆-akyl, poly-C₁-C₄-alkoxy-C₁-C₄-alkyl or represents optionally fluorine-, chlorine-, methyl-, ethyl-, n-propyl-, i-propyl-, n-butyl-, i-butyl-, tert-butyl-, methoxy-, ethoxy-, n-propoxy- or iso-propoxy-substituted C₃-C₆-cycloalkyl in which optionally one or two not directly adjacent methylene groups are replaced by oxygen and/or sulphur,
represents optionally fluorine-, chlorine-, bromine-, cyano-, nitro-, methyl-, ethyl-, n-propyl-, i-propyl-, methoxy-, ethoxy-, trifluoromethyl-, trifluoromethoxy-, methylthio-, ethylthio-, methylsulphonyl- or ethylsulphonyl-substituted phenyl, represents optionally fluorine-, chlorine-, bromine-, methyl-, ethyl-, n-propyl-, i-propyl-, methoxy-, ethoxy-, trifluoromethyl- or trifluoromethoxy-substituted benzyl,
represents in each case optionally fluorine-, chlorine-, bromine-, methyl- or ethyl-substituted furanyl, thienyl or pyridyl,
represents optionally fluorine-, chlorine-, methyl- or ethyl-substituted phenoxy-C₁-C₄-alkyl or
represents in each case optionally fluorine-, chlorine-, amino-, methyl- or ethyl-substituted pyridyloxy-C₁-C₄-alkyl, pyrimidyloxy-C₁-C₄-alkyl or thiazolyloxy-C₁-C₄-alkyl,
R² represents in each case optionally fluorine- or chlorine-substituted C₁-C₁₄-alkyl, C₂-C₁₄-alkenyl, C₁-C₄-alkoxy-C₂-C₆-alkyl or poly-C₁-C₄-alkoxy-C₂-C₆-alkyl,
represents optionally fluorine-, chlorine-, methyl-, ethyl-, n-propyl, iso-propyl- or methoxy-substituted C₃-C₆-cycloalkyl,
or represents in each case optionally fluorine-, chlorine-, cyano-, nitro-, methyl-, ethyl-, n-propyl-, i-propyl-, methoxy-, ethoxy-, trifluoromethyl- or trifluoromethoxy-substituted phenyl or benzyl,
R³ represents in each case optionally fluorine- or chlorine-substituted methyl, ethyl, propyl, iso-propyl, n-butyl, tert-butyl, or represents in each case optionally fluorine-, chlorine-, bromine-, methyl-, ethyl-, iso-propyl-, tert-butyl-, methoxy-, ethoxy-, iso-propoxy-, trifluoromethyl-, trifluoromethoxy-, cyano- or nitro-substituted phenyl or benzyl,
R⁴ and R⁵ independently of one another each represent in each case optionally fluorine- or chlorine-substituted C₁-C₄-alkyl, C₁-C₄-alkoxy, C₁-C₄-alkylamino, di-(C₁-C₄-alkyl)amino or C₁-C₄-alkylthio or represent in each case optionally fluorine-, chlorine-, bromine-, nitro-, cyano-, methyl-, methoxy-, trifluoromethyl- or trifluoromethoxy-substituted phenyl, phenoxy or phenylthio and
R⁶ and R⁷ independently of one another represent hydrogen, represent in each case optionally fluorine- or chlorine-substituted C₁-C₄-alkyl, C₃-C₆-cycloalkyl, C₁-C₄-alkoxy, C₃-C₄-alkenyl or C₁-C₄-alkoxy-C₂-C₄-alkyl, represent in each case optionally fluorine-, chlorine-, bromine-, methyl-, methoxy- or trifluoromethyl-substituted phenyl or benzyl, or together represent an optionally methyl- or ethyl-substituted C₅-C₆-alkylene radical in which optionally one methylene group is replaced by oxygen or sulphur.

4. Process for preparing compounds of the formula (I) according to Claims 1 to 3, **characterized in that**
(A) Compounds of the formula (I-1-a) 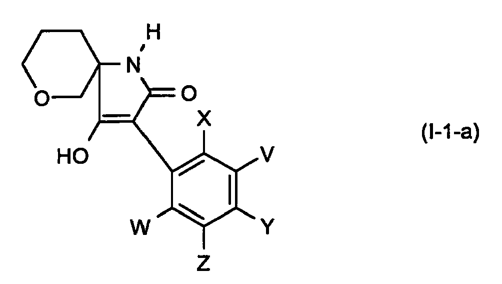 in which
V, W, X, Y and Z are each as defined in Claim 1
are obtained when
compounds of the formula (II) 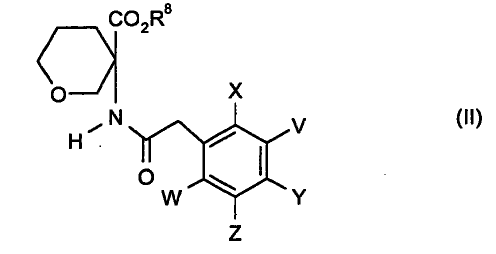 in which
V, W, X, Y and Z are each as defined above
and
R⁸ represents alkyl
are intramolecularly condensed in the presence of a diluent and in the presence of a base,
and the resulting compounds of the formula (I-I-a) shown above in which V, W, X, Y and Z are each as defined above are subsequently, if appropriate, in each case
(D)
α) reacted with acyl halides of the formula (V) 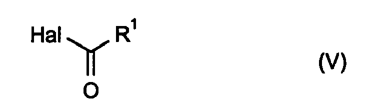 in which
R¹ is as defined in Claim 1 and
Hal represents halogen
or
β) with carboxylic anhydrides of the formula (VI)
R¹-CO-O-CO-R¹ (VI)
in which
R¹ is as defined above,
if appropriate in the presence of a diluent and if appropriate in the presence of an acid binder; or
(E) reacted with chloroformic esters or chloroformic thioesters of the formula (VII)
R²-M-CO-Cl (VII)
in which
R² and M are each as defined above,
if appropriate in the presence of a diluent and if appropriate in the presence of an acid binder;
(F) reacted with chloromonothioformic esters or chlorodithioformic esters of the formula (VIII) 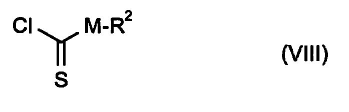 in which
M and R² are each as defined in Claim 1,
if appropriate in the presence of a diluent and if appropriate in the presence of an acid binder,
or
(G) reacted with sulphonyl chlorides of the formula (IX)
R³-SO₂-Cl (IX)
in which
R³ is as defined in Claim 1,
if appropriate in the presence of a diluent and if appropriate in the presence of an acid binder; or
(H) reacted with phosphorous compounds of the formula (X) 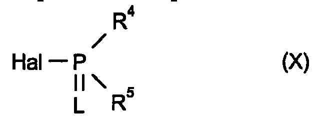 in which
L, R⁴ and R⁵ are each as defined in Claim 1 and
Hal represents halogen,
if appropriate in the presence of a diluent and if appropriate in the presence of an acid binder; or
(I) reacted with metal compounds or amines of the formulae (XI) or (XII)
Me(OR¹⁰)ₜ (XI) 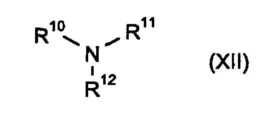
in which
Me represents a mono- or divalent metal
t represents the number 1 or 2 and
R¹⁰, R¹¹, R¹² independently of one another each represent hydrogen or alkyl,
if appropriate in the presence of a diluent,
or
(J)
α) reacted with isocyanates or isothiocyanates of the formula (XIII)
R⁶-N=C=L (XIII)
in which
R⁶ and L are each as defined in Claim 1,
if appropriate in the presence of a diluent and if appropriate in the presence of a catalyst or
β) reacted with carbamoyl chlorides or thiocarbamoyl chlorides of the formula (XIV) 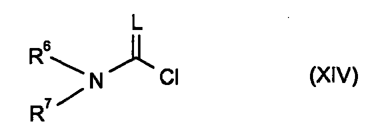
in which
L, R⁶ and R⁷ are each as defined in Claim 1,
if appropriate in the presence of a diluent and if appropriate in the presence of an acid binder.

5. Compounds of the formula (II) 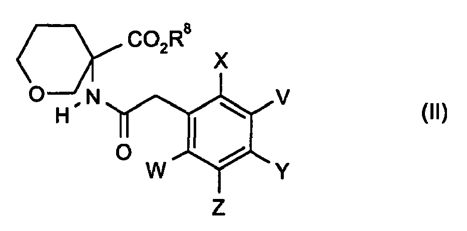 in which
V, W, X, Y and Z are each as defined in Claim 1 and
R⁸ represents alkyl.

6. Compounds of the formula (XVII) 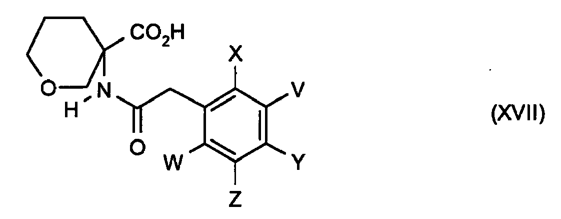 in which
V, W, X, Y and Z are each as defined in Claim 1.

7. Compounds of the formula (XXV) 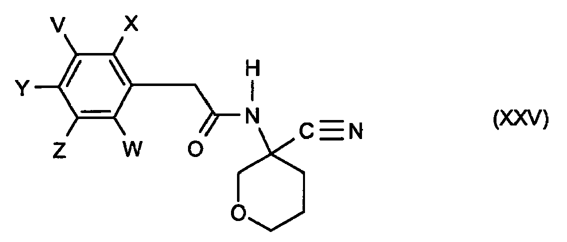 in which
V, W, X, Y and Z are each as defined in Claim 1.

8. Compounds of the formula (XXIV)

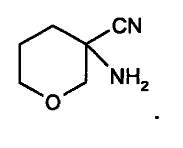

9. Compounds of the formula 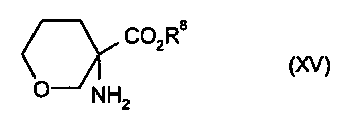 in which
R⁸ represents alkyl.

10. Compounds of the formula

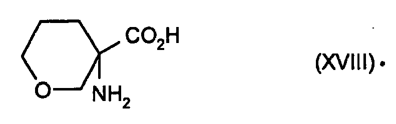

11. Pesticides or herbicides, **characterized in that** they contain a compound of the formula (I) according to Claims 1 to 3.

12. Use of compounds of the formula (I) according to Claims 1 to 3 for controlling pests and weeds, except for on the human or animal body.

13. Method for controlling pests and weeds, **characterized in that** compounds of the formula (I) according to Claims 1 to 3 are allowed to act on pests or weeds and/or their habitat, except for on the human or animal body.

14. Process for preparing pesticides and herbicides, **characterized in that** compounds of the formula (I) according to Claims 1 to 3 are mixed with extenders and/or surfactants.

## Revendications

1. Composés de formule (I) : 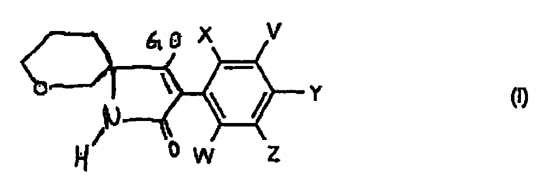 dans laquelle
V représente l'hydrogène, un halogène, un reste alkyle en C₁ à C₆ ou alkoxy en C₁ à C₆,
W représente l'hydrogène, un groupe nitro, cyano, un halogène, un reste alkyle en C₁ à C₆, alcényle en C₂ à C₆, alcynyle en C₂ à C₆, alkoxy en C₁ à C₆, halogénalkyle en C₁ à C₄, halogénalkoxy en C₁ à C₄ ou un reste phényle, phénoxy, phénylthio, phényl-(alkoxy en C₁ à C₄) ou phényl-(alkylthio en C₁ à C₄), chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
X représente un halogène, un reste alkyle en C₁ à C₆, alcényle en C₂ à C₆, alcynyle en C₂ à C₆, alkoxy en C₁ à C₆, halogénalkyle en C₁ à C₉, halogénalkoxy en C₁ à C₄, cyano, nitro, ou un reste phényle, phénoxy, phénylthio, phényl-(alkoxy en C₁ à C₄) ou phényl-(alkylthio en C₁ à C₄), chacun éventuellement substitué par un radical halogéno, alkyle en C₁ à C₆, alkoxy en C₁ à C₆, halogénalkyle en C₁ à C₄, halogénalkoxy en C₁ à C₄, nitro ou cyano,
Y représente l'hydrogène, un halogène, un reste alkyle en C₁ à C₆, alkoxy en C₁ à C₆, halogénalkyle en C₁ à C₄, halogénalkoxy en C₁ à C₄, cyano ou nitro,
Z représente l'hydrogène, un halogène, un reste alkyle en C₁ à C₆, alkoxy en C₁ à C₆, halogénalkyle en C₁ à C₄, halogénalkoxy en C₁ à C₄, hydroxy, cyano, nitro, ou un reste phénoxy, phénylthio, thiazolyloxy, pyridinyloxy, pyrimidyloxy, pyrazolyloxy, phényl-(alkyloxy en C₁ à C₄) ou phényl- (alkylthio en C₁ à C₄), chacun éventuellement substitué par un radical halogéno, alkyle en C₁ à C₄, alkoxy en C₁ à C₄, halogénalkyle en C₁ à C₄, halogénalkoxy en C₁ à C₄, nitro ou cyano, ou bien
G représente l'hydrogène (a) ou l'un des groupes : 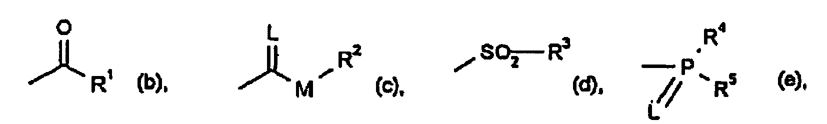 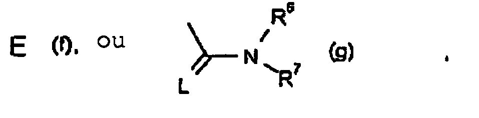 dans lesquels
E représente un équivalent d'ion métallique ou un ion ammonium,
L représente l'oxygène ou le soufre, et
M représente l'oxygène ou le soufre,
R¹ représente un reste alkyle en C₁ à C₂₀, alcényle en C₂ à C₂₀, (alkoxy en C₁ à C₈)-(alkyle en C₁ à C₈), (alkylthio en C₁ à C₈) - (alkyle en C₁ à C₈) ou poly(alkoxy en C₁ à C₈) - (alkyle en C₁ à C₈), chacun éventuellement substitué par un radical halogéno ou cyano, ou bien un reste cycloalkyle en C₃ à C₈ éventuellement substitué par un radical halogéno, alkyle en C₁ à C₆ ou alkoxy en C₁ à C₆ et dans lequel, le cas échéant, un groupe méthylène ou deux groupes méthylène non contigus sont remplacés par de l'oxygène et/ou du soufre,
un reste phényle éventuellement substitué par un radical halogéno, cyano, nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyle en C₁ à C₆,
un reste phényl-(alkyle en C₁ à C₆) éventuellement substitué par un radical halogéno, nitro, cyano, alkyle en C₁ à C₆, alkoxy en C₁ à C₆, halogénalkyle en C₁ à C₆ ou halogénalkoxy en C₁ à C₆,
un reste hétaryle pentagonal ou hexagonal éventuellement substitué par un radical halogéno ou alkyle en C₁ à C₆, ayant un ou deux hétéroatomes de la série oxygène, soufre et azote,
un reste phénoxy-(alkyle en C₁ à C₆) éventuellement substitué par un radical halogéno ou alkyle en C₁ à C₆, ou bien
un reste hétaryloxy-(alkyle en C₁ à C₆) pentagonal ou hexagonal éventuellement substitué par un radical halogéno, amino ou alkyle en C₁ à C₆, ayant un ou deux hétéroatomes de la série oxygène, soufre et azote,
R² représente un reste alkyle en C₁ à C₂₀, alcényle en C₂ à C₂₀, (alkoxy en C₁ à C₈)-(alkyle en C₂ à C₈) ou poly(alkoxy en C₁ à C₈) - (alkyle en C₂ à C₈), chacun éventuellement substitué par un radical halogéno ou cyano,
un reste cycloalkyle en C₃ à C₈ éventuellement substitué par un radical halogéno, alkyle en C₁ à C₆ ou alkoxy en C₁ à C₆, ou bien
un reste phényle ou benzyle, chacun éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ représente un reste alkyle en C₁ à C₈ éventuellement substitué par un halogène, ou un reste phényle ou benzyle, chacun éventuellement substitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ représentent, indépendamment l'un de l'autre, un reste alkyle en C₁ à C₈, alkoxy en C₁ à C₈, alkylamino en C₁ à C₈, di (alkyle en C₁ à C₈)amino, alkylthio en C₁ à C₈ ou alcénylthio en C₃ à C₈, chacun éventuellement substitué par un halogène, ou bien un reste phényle, phénoxy ou phénylthio, chacun éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄, et
R⁶ et R⁷ représentent, indépendamment l'un de l'autre, l'hydrogène, un reste alkyle en C₁ à C₈, cycloalkyle en C₃ à C₈, alkoxy en C₁ à C₈, alcényle en C₃ à C₈ ou (alkoxy en C₁ à C₈) - (alkyle en C₂ à C₈), chacun éventuellement substitué par un radical halogéno ou cyano, un reste phényle ou benzyle, chacun éventuellement substitué par un radical halogéno, alkyle en C₁ à C₈, halogénalkyle en C₁ à C₈ ou alkoxy en C₁ à C₈, ou forment conjointement un reste alkylène en C₃ à C₆ éventuellement substitué par un radical alkyle en C₁ à C₆ et dans lequel, le cas échéant, un groupe méthylène est remplacé par l'oxygène ou le soufre.

2. Composés de formule (I) suivant la revendication 1, formule dans laquelle
V représente l'hydrogène, le fluor, le chlore, le brome, un reste alkyle en C₁ à C₄ ou alkoxy en C₁ à C₄,
W représente l'hydrogène, un groupe nitro, cyano, le fluor, le chlore, le brome, un reste alkyle en C₁ à C₄, alkoxy en C₁ à C₄, halogénalkyle en C₁ ou C₂, halogénalkoxy en C₁ ou C₂ ou un reste phényle, phénoxy ou benzyloxy, chacun éventuellement substitué par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano,
X représente le fluor, le chlore, le brome, un reste alkyle en C₁ à C₄, alkoxy en C₁ à C₄, halogénalkyle en C₁ ou C₂, halogénalkoxy en C₁ ou C₂, cyano, nitro, ou un reste phényle, phénoxy ou benzyloxy, chacun éventuellement substitué par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano,
Y représente l'hydrogène, le fluor, le chlore, le brome, un reste alkyle en C₁ à C₄, alkoxy en C₁ à C₄, halogénalkyle en C₁ ou C₂, halogénalkoxy en C₁ ou C₂, cyano ou nitro,
Z représente l'hydrogène, le fluor, le chlore, le brome, un reste alkyle en C₁ à C₉, alkoxy en C₁ à C₄, halogénalkyle en C₁ ou C₂, halogénalkoxy en C₁ ou C₂, hydroxy, cyano, nitro, ou un reste phénoxy ou benzyloxy, chacun éventuellement substitué par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano,
G représente l'hydrogène (a) ou l'un des groupes : 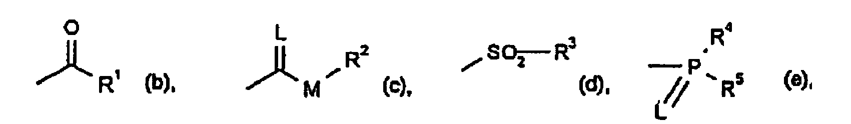 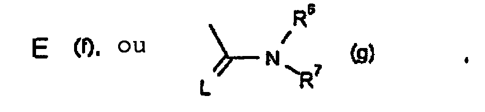 dans lesquels
E représente un équivalent d'ion métallique ou un ion ammonium,
L représente l'oxygène ou le soufre, et
M représente l'oxygène ou le soufre,
R¹ représente un reste alkyle en C₁ à C₁₆, alcényle en C₂ à C₁₆, (alkoxy en C₁ à C₆) - (alkyle en C₁ à C₆), (alkylthio en C₁ à C₆)-(alkyle en C₁ à C₆) ou poly (alkoxy en C₁ à C₆) - (alkyle en C₁ à C₆), chacun éventuellement substitué par du fluor ou par du chlore, ou un reste cycloalkyle en C₃ à C₇ éventuellement substitué par un radical fluoro, chloro, alkyle en C₁ à C₅ ou alkoxy en C₁ à C₅ et dans lequel, le cas échéant, un groupe méthylène ou deux groupes méthylène non contigus sont remplacés par de l'oxygène et/ou du soufre,
un reste phényle éventuellement substitué par un radical fluoro, chloro, bromo, cyano, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄,
un reste phényl-(alkyle en C₁ à C₄) éventuellement substitué par un radical fluoro, chloro, bromo, alkyle en C₁ à C₄, alkoxy en C₁ à C₄, halogénalkyle en C₁ à C₃ ou halogénalkoxy en C₁ à C₃,
un reste pyrazolyle, thiazolyle, pyridyle, pyrimidyle, furannyle ou thiényle, chacun éventuellement substitué par un radical fluoro, chloro, bromo ou alkyle en C₁ à C₄,
un reste phénoxy-(alkyle en C₁ à C₅) éventuellement substitué par un radical fluoro, chloro, bromo ou alkyle en C₁ à C₄, ou bien
un reste pyridyloxy-(alkyle en C₁ à C₅), pyrimidyloxy-(alkyle en C₁ à C₅) ou thiazolyloxy-(alkyle en C₁ à C₅), chacun éventuellement substitué par un radical fluoro, chloro, bromo, amino ou alkyle en C₁ à C₄,
R² représente un reste alkyle en C₁ à C₁₆, alcényle en C₂ à C₁₆, (alkoxy en C₁ à C₆) - (alkyle en C₂ à C₆) ou poly (alkoxy en C₁ à C₆) - (alkyle en C₂ à C₆), chacun éventuellement substitué par du fluor ou par du chlore,
un reste cycloalkyle en C₃ à C₇ éventuellement substitué par un radical fluoro, chloro, alkyle en C₁ à C₄ ou alkoxy en C₁ à C₄, ou bien
un reste phényle ou benzyle, chacun éventuellement substitué par un radical fluoro, chloro, bromo, cyano, nitro, alkyle en C₁ à C₄, alkoxy en C₁ à C₃, halogénalkyle en C₁ à C₃ ou halogénalkoxy en C₁ à C₃,
R³ représente un reste alkyle en C₁ à C₆ éventuellement substitué par du fluor ou par du chlore, ou un reste phényle ou benzyle, chacun éventuellement substitué par un radical fluoro, chloro, bromo, alkyle en C₁ à C₄, alkoxy en C₁ à C₄, halogénalkoxy en C₁ ou C₂, halogénalkyle en C₁ ou C₂, cyano ou nitro,
R⁴ et R⁵ représentent, indépendamment l'un de l'autre, un reste alkyle en C₁ à C₆, alkoxy en C₁ à C₆, alkylamino en C₁ à C₆, di (alkyle en C₁ à C₆) amino, alkylthio en C₁ à C₆ ou alcénylthio en C₃ ou C₄, chacun éventuellement substitué par du fluor ou par du chlore, ou un reste phényle, phénoxy ou phénylthio, chacun éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃, et
R⁶ et R⁷ représentent, indépendamment l'un de l'autre, l'hydrogène, un reste alkyle en C₁ à C₆, cycloalkyle en C₃ à C₆, alkoxy en C₁ à C₆, alcényle en C₃ à C₆ ou (alkoxy en C₁ à C₆)-(alkyle en C₂ à C₆), chacun éventuellement substitué par du fluor ou par du chlore, un reste phényle ou benzyle, chacun éventuellement substitué par un radical fluoro, chloro, bromo, halogénalkyle en C₁ à C₅, alkyle en C₁ à C₅ ou alkoxy en C₁ à C₅, ou forment conjointement un reste alkylène en C₃ à C₆ éventuellement substitué par un radical alkyle en C₁ à C₄ et dans lequel, le cas échéant, un groupe méthylène est remplacé par de l'oxygène ou par du soufre.

3. Composés de formule (I) suivant la revendication 1, formule dans laquelle
V représente l'hydrogène, le fluor, le chlore, le brome, un reste méthyle, éthyle, propyle, isopropyle, tertiobutyle, méthoxy, éthoxy, propoxy ou isopropoxy,
W représente l'hydrogène, un groupe nitro, cyano, le fluor, le chlore, le brome, un reste méthyle, éthyle, propyle, n-butyle, isopropyle, isobutyle, méthoxy, éthoxy, propoxy, isopropoxy, trifluorométhyle, difluorométhoxy, trifluorométhoxy, phényle ou benzyloxy,
X représente le fluor, le chlore, le brome, un reste méthyle, éthyle, propyle, butyle, isobutyle, isopropyle, méthoxy, éthoxy, propoxy, isopropoxy, trifluorométhyle, trifluorométhoxy, difluorométhoxy, cyano, nitro, phényle ou benzyloxy,
Y représente l'hydrogène, le fluor, le chlore, le brome, un reste méthyle, éthyle, propyle, isopropyle, n-butyle, isobutyle, tertiobutyle, méthoxy, éthoxy, propoxy, isopropoxy, trifluorométhyle, trifluorométhoxy, difluorométhoxy, cyano ou nitro,
Z représente l'hydrogène, le fluor, le chlore, le brome, un reste méthyle, éthyle, propyle, butyle, isobutyle, isopropyle, tertiobutyle, méthoxy, éthoxy, propoxy, isopropoxy, trifluorométhyle, trifluorométhoxy, difluorométhoxy, cyano ou nitro, ou bien
G représente l'hydrogène (a) ou l'un des groupes: 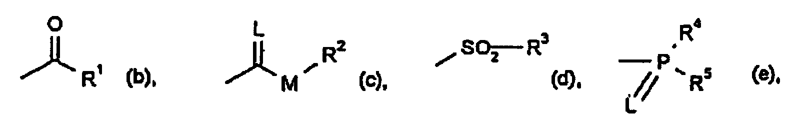 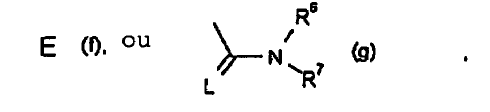 dans lesquels
E représente un équivalent d'ion métallique ou un ion ammonium,
L représente l'oxygène ou le soufre, et
M représente l'oxygène ou le soufre,
R¹ représente un reste alkyle en C₁ à C₁₄, alcényle en C₂ à C₁₄, (alkoxy en C₁ à C₄) - (alkyle en C₁ à C₆), (alkylthio en C₁ à C₄)-(alkyle en C₁ à C₆), poly(alkoxy en C₁ à C₄)-(alkyle en C₁ à C₄), chacun éventuellement substitué par du fluor ou par du chlore, ou un reste cycloalkyle en C₃ à C₆ éventuellement substitué par un radical fluoro, chloro, méthyle, éthyle, n-propyle, isopropyle, n-butyle, isobutyle, tertiobutyle, méthoxy, éthoxy, n-propoxy ou isopropoxy, dans lequel, le cas échéant, un groupe méthylène ou deux groupes méthylène non contigus sont remplacés par de l'oxygène et/ou par du soufre,
un reste phényle éventuellement substitué par un radical fluoro, chloro, bromo, cyano, nitro, méthyle, éthyle, n-propyle, isopropyle, méthoxy, éthoxy, trifluorométhyle, trifluorométhoxy, méthylthio, éthylthio, méthylsulfonyle ou éthylsulfonyle,
un reste benzyle éventuellement substitué par un radical fluoro, chloro, bromo, méthyle, éthyle, n-propyle, isopropyle, méthoxy, éthoxy, trifluorométhyle ou trifluorométhoxy,
un reste furannyle, thiényle ou pyridyle, chacun éventuellement substitué par un radical fluoro, chloro, bromo, méthyle ou éthyle,
un reste phénoxy-(alkyle en C₁ à C₄) éventuellement substitué par un radical fluoro, chloro, méthyle ou éthyle, ou bien
un reste pyridyloxy-(alkyle en C₁ à C₄), pyrimidyloxy-(alkyle en C₁ à C₄) ou thiazolyloxy- (alkyle en C₁ à C₄), chacun éventuellement substitué par un radical fluoro, chloro, amino, méthyle ou éthyle,
R² représente un reste alkyle en C₁ à C₁₄, alcényle en C₂ à C₁₄, (alkoxy en C₁ à C₄)-(alkyle en C₂ à C₆) ou poly(alkoxy en C₁ à C₄)-(alkyle en C₂ à C₆), chacun éventuellement substitué par du fluor ou par du chlore,
un reste cycloalkyle en C₃ à C₆ éventuellement substitué par un radical fluoro, chloro, méthyle, éthyle, n-propyle, isopropyle ou méthoxy,
ou bien un reste phényle ou benzyle, chacun éventuellement substitué par un radical fluoro, chloro, cyano, nitro, méthyle, éthyle, n-propyle, isopropyle, méthoxy, éthoxy, trifluorométhyle ou trifluorométhoxy,
R³ représente un reste méthyle, éthyle, propyle, isopropyle, n-butyle, tertiobutyle, chacun éventuellement substitué par du fluor ou par du chlore, ou un reste phényle ou benzyle, chacun éventuellement substitué par un radical fluoro, chloro, bromo, méthyle, éthyle, isopropyle, tertiobutyle, méthoxy, éthoxy, isopropoxy, trifluorométhyle, trifluorométhoxy, cyano ou nitro,
R⁴ et R⁵ représentent, indépendamment l'un de l'autre, un reste alkyle en C₁ à C₄, alkoxy en C₁ à C₄, alkylamino en C₁ à C₄, di (alkyle en C₁ à C₄) amino ou alkylthio en C₁ à C₄, chacun éventuellement substitué par du fluor ou par du chlore, ou un reste phényle, phénoxy ou phénylthio, chacun éventuellement substitué par un radical fluoro, chloro, bromo, nitro, cyano, méthyle, méthoxy, trifluorométhyle ou trifluorométhoxy, et
R⁶ et R⁷ représentent, indépendamment l'un de l'autre, l'hydrogène, un reste alkyle en C₁ à C₄, cycloalkyle en C₃ à C₆, alkoxy en C₁ à C₄, alcényle en C₃ ou C₄ ou (alkoxy en C₁ à C₄)-(alkyle en C₂ à C₄), chacun éventuellement substitué par du fluor ou par du chlore, un reste phényle ou benzyle, chacun éventuellement substitué par un radical fluoro, chloro, bromo, méthyle, méthoxy ou trifluorométhyle, ou forment ensemble un reste alkylène en C₅ ou C₆ éventuellement substitué par un radical méthyle ou éthyle et dans lequel, le cas échéant, un groupe méthylène est remplacé par de l'oxygène ou par du soufre.

4. Procédé de production de composés de formule (I) suivant la revendication 1-3, **caractérisé en ce que** (A) on obtient des composés de formule (I-1-a) 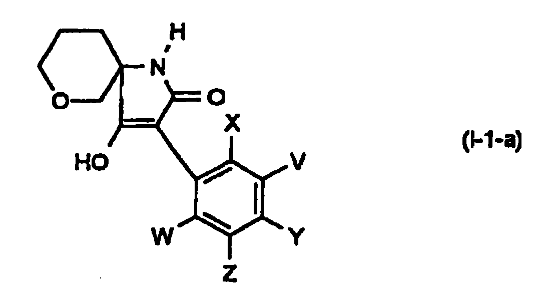 dans laquelle
V, W, X, Y et Z ont les définitions indiquées dans la revendication 1,
lorsqu'on effectue la condensation intramoléculaire de composés de formule (II) : 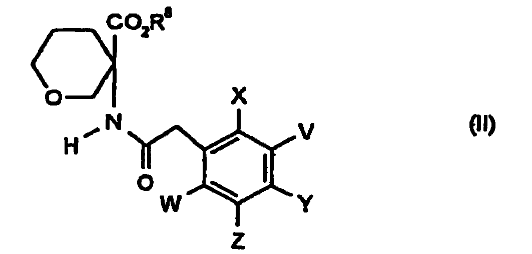 dans laquelle
V, W, X, Y et Z ont les définitions indiquées ci-dessus, et
R⁸ représente un reste alkyle,
en présence d'un diluant et en présence d'une base,
puis, le cas échéant, les composés ainsi obtenus de formule (I-1-a) indiquée ci-dessus, dans laquelle V, W, X, Y et Z ont les définitions indiquées ci-dessus, sont amenés à réagir respectivement
(D)
α) avec des halogénures d'acides de formule (V) : 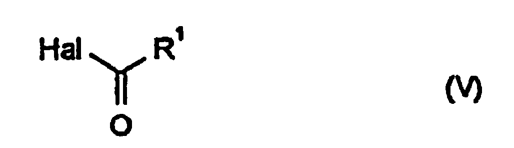 dans laquelle
R¹ a la définition indiquée dans la revendication 1 et
Hal représente un halogène
ou bien
β) avec des anhydrides d'acides carboxyliques de formule (VI) :
R¹-CO-O-CO R¹ (VI)
dans laquelle
R¹ a la définition indiquée ci-dessus,
éventuellement en présence d'un diluant et, le cas échéant, en présence d'un accepteur d'acide, ou bien
(E) avec des esters d'acide chloroformique ou des thioesters d'acide chloroformique de formule (VII) :
R²-M-CO-Cl (VII)
dans laquelle
R² et M ont les définitions indiquées ci-dessus,
éventuellement en présence d'un diluant et, le cas échéant, en présence d'un accepteur d'acide,
(F) avec des esters d'acide chloromonothioformique ou des esters d'acide chlorodithioformique de formule (VIII) : 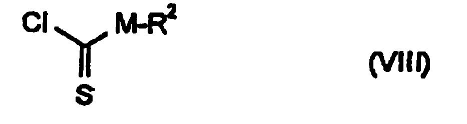 dans laquelle
M et R² ont les définitions indiquées dans la revendication 1,
éventuellement en présence d'un diluant et, le cas échéant, en présence d'un accepteur d'acide,
ou bien
(G) avec des chlorures d'acides sulfoniques de formule (IX) :
R³-SO₂-Cl (IX)
dans laquelle
R³ a la définition indiquée dans la revendication 1,
éventuellement en présence d'un diluant et, le cas échéant, en présence d'un accepteur d'acide ; ou bien
(H) avec des composés phosphorés de formule (X) : 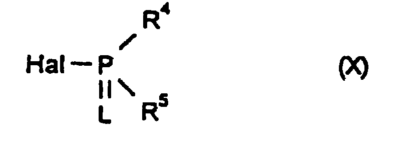 dans laquelle
L, R⁴ et R⁵ ont les définitions indiquées dans la revendication 1 et
Hal représente un halogène,
éventuellement en présence d'un diluant et, le cas échéant, en présence d'un accepteur d'acide ; ou bien
(I) avec des composés métalliques ou des amines de formule (XI) ou (XII) :
Me(OR¹⁰)ₜ (XI) 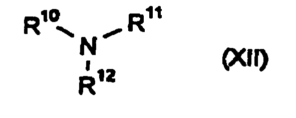
dans lesquelles
Me représente un métal monovalent ou divalent,
t représente le nombre 1 ou 2, et
R¹⁰, R¹¹, R¹² représentent, indépendamment les uns des autres, l'hydrogène ou un reste alkyle,
éventuellement en présence d'un diluant,
ou bien
(J)
α) avec des isocyanates ou des isothiocyanates de formule (XIII) :
R⁶-N=C=L (XIII)
dans laquelle
R⁶ et L ont les définitions indiquées dans la revendication 1,
éventuellement en présence d'un diluant et, le cas échéant, en présence d'un catalyseur, ou bien
β) avec des chlorures d'acides carbamiques ou des chlorures d'acides thiocarbamiques de formule (XIV) : 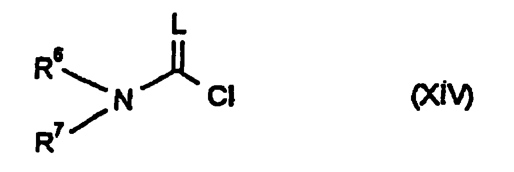
dans laquelle
L, R⁶ et R⁷ ont les définitions indiquées dans la revendication 1,
éventuellement en présence d'un diluant et, le cas échéant, en présence d'un accepteur d'acide.

5. Composés de formule (II) : 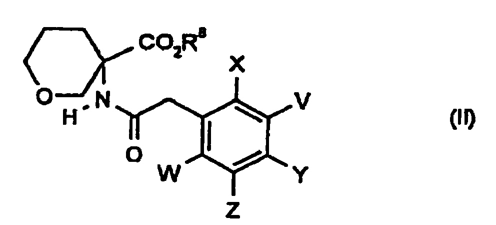 dans laquelle
V, W, X, Y et Z ont les définitions indiquées dans la revendication 1, et
R⁸ est un reste alkyle.

6. Composés de formule (XVII) : 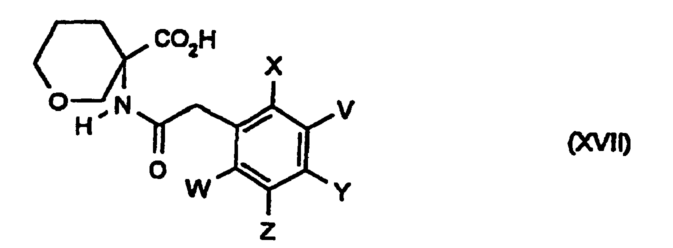 dans laquelle
V, W, X, Y et Z ont les définitions indiquées dans la revendication 1.

7. Composés de formule (XXV) : 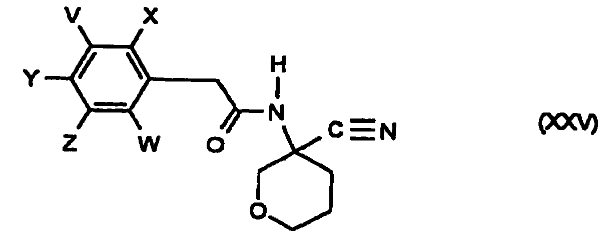 dans laquelle
V, W, X, Y et Z ont les définitions indiquées dans la revendication 1.

8. Composé de formule (XXIV) :

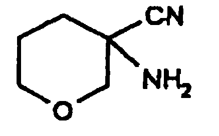

9. Composés de formule : 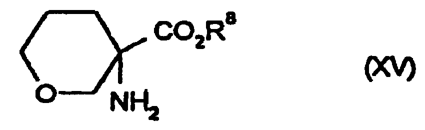 dans laquelle
R⁸ est un reste alkyle.

10. Composé de formule :

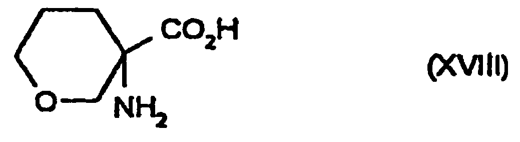

11. Pesticides ou herbicides, **caractérisés par** une teneur en un composé de formule (I) suivant la revendication 1-3.

12. Utilisation de composés de formule (I) suivant la revendication 1-3, pour combattre des parasites et des mauvaises herbes, excepté sur le corps humain ou le corps d'un animal.

13. Procédé pour combattre des parasites et des mauvaises herbes, **caractérisé en ce qu'**on fait agir des composés de formule (I) suivant la revendication 1-3 sur des parasites ou des mauvaises herbes et/ou sur leur milieu, excepté sur le corps humain ou le corps d'un animal.

14. Procédé de préparation de pesticides et d'herbicides, **caractérisé en ce qu'**on mélange des composés de formule (I) suivant la revendication 1-3 avec des diluants et/ou des agents tensioactifs.
